(19) Europäisches Patentamt
European Patent Office
Office européen des brevets

(11) Veröffentlichungsnummer: **0 470 617 A2**

(12) **EUROPÄISCHE PATENTANMELDUNG**

(21) Anmeldenummer: **91113338.7**

(22) Anmeldetag: **08.08.91**

(51) Int. Cl.[5]: **C07J 31/00**, C07J 5/00, C07J 7/00, A61K 31/57, //C07J21/00

(30) Priorität: **10.08.90 DE 4025342**

(43) Veröffentlichungstag der Anmeldung: **12.02.92 Patentblatt 92/07**

(84) Benannte Vertragsstaaten: **AT BE CH DE DK ES FR GB GR IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Stache, Ulrich, Dr.**
**Goldgrabenstrasse 20**
**W-6238 Hofheim/Ts.(DE)**
Erfinder: **Dürckheimer, Walter, Dr.**
**Im Lerchenfeld 45**
**W-6234 Hattersheim a.M.(DE)**
Erfinder: **Alpermann, Hans Georg, Dr.**
**Am Eichkopf 10**
**W-6240 Königstein/Ts.(DE)**
Erfinder: **Petri, Walter, Dr.**
**Walkmühlstrasse 62**
**W-6200 Wiesbaden(DE)**

(54) **In 17-Stellung substituierte Corticoid-17-alkylcarbonate, Verfahren zu deren Herstellung und diese enthaltende Arzneimittel.**

(57) Corticoid-17-alkylcarbonate der Formel I

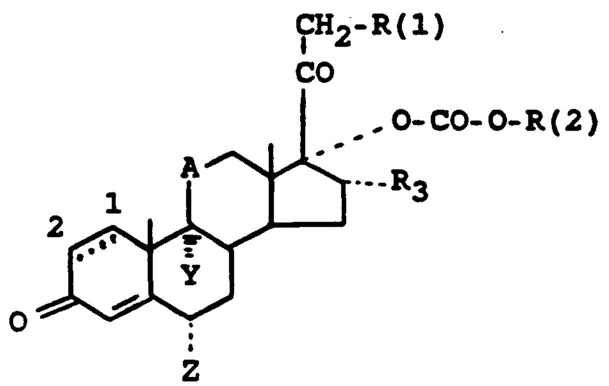

I,

mit A = CHOH in beliebiger sterischer Anordnung, C=O, $CH_2$; Y = H, F, Cl; Z = H, F, $CH_3$; R(1) = Hal, O-Acyl, Carbonylalkyl, Sulfonsäurealkylester, Sulfonsäurearylester; R(2) = verzweigtes Alkyl oder $(CH_2)_{2-4}$-$OCH_3$ und
R(3) = H, Methyl
haben hervorragende lokale und topische antiphlogistische Wirkung. Sie zeichnen sich durch ein besonders gutes Verhältnis von lokaler zu systemischer antiinflammatorischer Wirksamkeit aus und zeigen auch z.T. stärkere lokale antiphlogistische Wirksamkeiten als ihre isomeren Corticoid-17-alkylcarbonate mit einer linearen Alkylgruppe im 17-Alkycarbonat-Teil.

EP 0 470 617 A2

Die Erfindung betrifft Corticoid-17-alkylcarbonate der Formel I

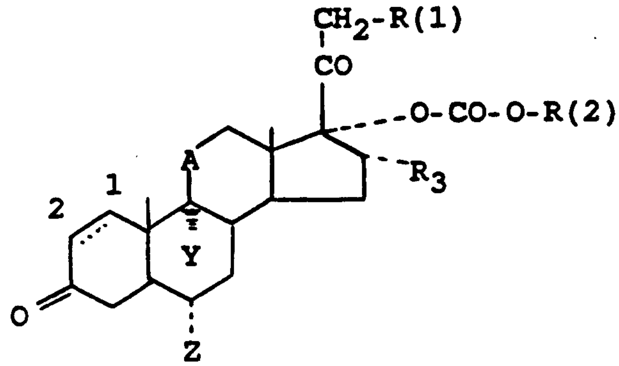

(I),

in welcher bedeuten:

A = CHOH in beliebiger sterischer Anordnung, $CH_2$, C=O,

Y = Wasserstoff, Fluor, Chlor,

Z = Wasserstoff, Fluor, Methyl

R(1) = F, Cl, Br, J, O-Acyl der Formel II: $-O-CO-(CH_2)_n-R(4)$, Oxycarbonyloxyalkyl der Formel III: $-O-CO-O-(CH_2)_n-R(4)$ Sulfonsäurealkyl-, Sulfonsäurearylester der Formel IV: $-O-SO_2-R(5)$
mit R(4) gleich Wasserstoff, ($C_1$-$C_{10}$)-Alkyl, ($C_3$-$C_6$)-Cycloalkyl oder, wenn n = 1 ist, Fluor, Chlor, Brom,
R(5) gleich ($C_1$-$C_4$)-Alkyl, Phenyl, Chlorphenyl, Methylphenyl,
n eine ganze Zahl von 0 bis 4,

R(2) = verzweigtes ($C_3$-$C_8$)-Alkyl, $-(CH_2)_{2-4}-OCH_3$

R(3) = Wasserstoff, $\alpha$-Methyl.

Bevorzugt sind Verbindungen der Formel I mit

R(1) A, Y, Z und R(3) wie angegeben und

R(2) gleich verzweigtes ($C_3$-$C_5$)-Alkyl und $-(CH_2)_2-OCH_3$,

R(5) gleich Methyl, Ethyl, Propyl, Phenyl, das unsubstituiert ist oder in p-Stellung Chlor- oder Methylsubstituiert ist.

Ganz besonders bevorzugt sind Verbindungen I mit

Y gleich Wasserstoff und

Z gleich Wasserstoff oder Methyl, sowie

R(2) gleich $-CH(CH_3)_2$, $-CH_2-CH(CH_3)_2$, $-CH_2C(CH_3)_3$, $-(CH_2)_2-O-CH_3$;

die Bindung zwischen $C_1$- und $C_2$ ist eine Einfach- oder eine Doppelbindung, wie durch die punktierte Linie in Formel I angegeben.

Die Erfindung betrifft desweiteren auch ein Verfahren zur Herstellung von Verbindungen I, welches dadurch gekennzeichnet ist, daß man Verbindungen der Formel V

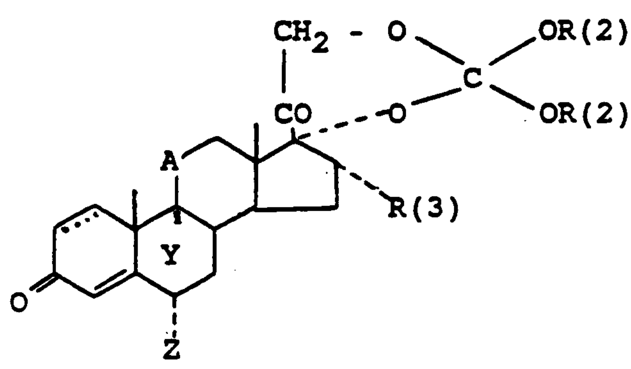

(V)

mit einer schwachen Säure hydrolysiert und die erhaltene 21-Hydroxy-Verbindung mit einem Halogenid oder Anhydrid einer Carbonsäure der Formel VI

R(4)-$(CH_2)_n$-COOH VI

oder einem Halogenformat der Formel VII

R(4)-$(CH_2)_n$-OCO-Halogen

oder einem Sulfonsäurehalogenid der Formel VIII

R(5)-$SO_2$-Halogen VIII

verestert
und gegebenenfalls den so erhaltenen 21-Sulfonsäureester mit Halogenidsalzen zu 21-Halogeniden der Formel I mit R(1) gleich Chlor, Brom, Jod, Fluor umsetzt.

Gegenstand der Erfindung ist weiterhin ein Verfahren zur Herstellung der Verbindungen der Formel V, bei dem man die Basis-Corticosteroide der Formel VI

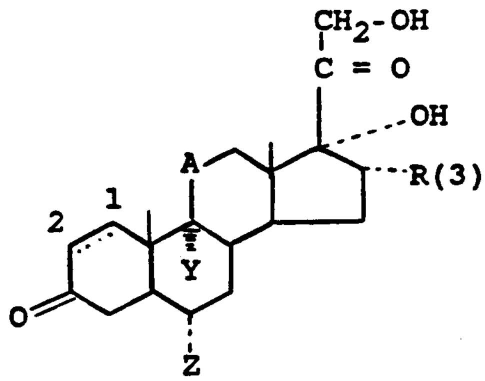

(VI)

mit im Alkylanteil verzweigten oder substituierten Tetraalkyl-orthocarbonaten der Formel VII

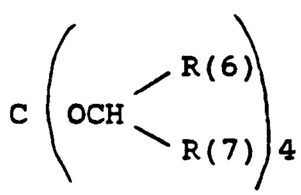

(VII)

in inerten Lösungsmitteln bei Temperaturen von größer als 20° bis 120 °C, vorzugsweise bis zum Siedepunkt der Reaktionsgemische, insbesondere bei etwa 50° bis 60 °C, umsetzt.

In den Formeln VI haben A, Y, Z, und R(3) die oben angegebenen Bedeutungen,

R(6) = H, $CH_3$ und

R(7) = $CH_3$, $CH(CH_3)_2$, $C(CH_3)_3$ oder $CH_2OCH_3$.

Ausgeschlossen ist die Herstellung von Verbindungen, bei denen gleichzeitig R(6) = H ist und R(7) keine verzweigte Kohlenstoffkette enthält.

Die als Ausgangssubstanzen benötigten Steroid-17,21-dialkylorthocarbonate der Formel V werden in Anlehnung an das Verfahren nach der deutschen Patentschrift Nr. 16 68 079 hergestellt. Allerdings sind zur Herstellung der im Alkylrest verzweigten und durch Alkoxygruppen substituierten 17,21-Dialkylorthocarbonate der Formel V in der Regel wesentlich höhere Reaktionstemperaturen, meistens größer als 50 °C, und längere Reaktionszeiten (doppelt bis vierfache Reaktionszeiten) erforderlich als es bei den linearen Analogen gemäß der deutschen Patentschrift 16 68 079 (HOE 68/F 012) der Fall ist.

Die 21-Hydroxygruppe kann, je nachdem ob ein 21-Alkylcarbonat, ein 21-Carbonsäurederivat oder ein 21-Alkyl- oder -Arylsulfonsäureester der zugrundeliegenden Corticoid-17-alkylcarbonate hergestellt werden soll, mit den dazu üblichen Acylierungsmitteln umgesetzt werden:

a) Zur Herstellung von 21-Alkylcarbonaten werden vorzugsweise Chlorameisensäurealkylester der Formel

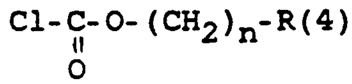

verwendet, in der R(4) die zur Formel I angegebene Bedeutung hat. Vorzugsweise wird Chlorameisensäure-methylester, -ethylester, -propylester ode -butylester verwendet.

b) Zur Herstellung von 21-Carbonsäureestern werden vorzugsweise entweder Carbonsäurehalogenide der Formel

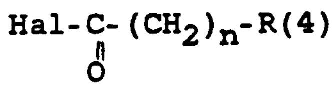 ,

in der Hal Cl, Br oder J darstellt und R(4) die zur Formel I angegebene Bedeutung hat, oder Carbonsäureanhydride der Formel $[OC-(CH_2)_n-R(4)]_2O$, in der R(4) die zur Formel I angegebene Bedeutung hat, verwendet. Beispielsweise können verwendet werden: Essigsäure-, Propionsäure-, Buttersäure-, Valeriansäure-chlorid oder -anhydrid, Cyclopropansäure-, Cyclopentylpropionsäure- oder Önanthsäurechlorid.

c) Zur Herstellung von 21-Sulfonsäureestern kommen Sulfonsäurehalogenide der Formel $Cl-SO_2-R(5)$ in der R(5) die zur Formel I angegebene Bedeutung hat, in Frage.

Vorzugsweise werden Methansulfonsäure- oder p-Chlorphenylsulfonsäurechlorid oder p-Toluolsulfonsäurechlorid eingesetzt.

d) Die erhaltenen Corticoid-17-alkylcarbonat-21-sulfonsäureester können gegebenenfalls mit Halogenidsalzen in inerten Lösungsmitteln gemäß der Europäischen Patentschrift 00 04 975 zu den entsprechenden Corticoid-17-alkylcarbonat-21-halogeniden umgesetzt werden.

Für die zweite Verfahrensstufe löst, man die Steroidkomponente in einem inerten Lösungsmittel, beispielsweise in einem Ether, wie Dioxan, Tetrahydrofuran, Diglym, oder gegebenenfalls halogenierten Kohlenwasserstoffen, wie Benzol, Toluol, Cyclohexan, Methylenchlorid, Chloroform oder in einem Gemisch dieser Lösungsmittel. Zur Entfernung der in der Reaktion entstehenden Halogenwasserstoffsäure setzt man 1-1000 Molequivalente einer tertiären Base, wie Pyridin, Chinolin, Triethylamin oder Dimethylanilin zu. Man kann aber auch eine anorganische Base wie Natriumhydrogencarbonat oder Calciumcarbonat zur Entfernung der Säure benutzen.

Anschließend tropft man 1-200 Molequivalente, vorzugsweise 1-3 Molequivalente eines der oben angeführten Acylierungsmittel, gegebenenfalls gelöst in einem der oben angeführten Lösungsmittel, bei einer Temperatur von -40 °C bis zum Siedepunkt des verwendeten Lösungsmittels, vorzugsweise von 0 °C bis 25 °C, zu. Anschließend läßt man das Reaktionsgemisch eine bis 120 Stunden bei einer Temperatur von -40 °C bis zum Siedepunkt des Lösungsmittels, vorzugsweise von 0 °C bis 25 °C stehen.

Bei Verwendung von Carbonsäureanhydriden als Acylierungsmittel ist es oft von Vorteil, ohne Zusatz von Lösungsmitteln zu arbeiten. Es reicht in der Regel aus, lediglich die organische Base, vorzugsweise Pyridin, dem im Überschuß angewandten Säureanhydrid zuzufügen.

Zur Aufarbeitung gießt man das Reaktionsgemisch in Wasser, das gegebenenfalls mit Natriumbicarbonat versetzt wurde, wobei die Reaktionsprodukte, oft erst nach längerem Stehen, im allgemeinen kristallin ausfallen. Ölig gebliebene Reaktionsprodukte werden durch Ausschütteln mit einem geeigneten Extraktionsmittel und Eindampfen angereichert. Die Reaktionsprodukte können, falls erforderlich, durch Umkristallisieren oder durch Chromatographie aufgetrennt oder gereinigt werden. Oft genügt auch intensives Digerieren in einem das Reaktionsprodukt möglichst wenig oder nicht lösenden organischen Lösungsmittel, wie Diethylether oder Cyclohexan oder einem Gemisch aus diesen Komponenten, zur weiteren Reinigung der Reaktionsprodukte.

Eine Hydroxygruppe in 11-Stellung kann gegebenenfalls nach üblichen Methoden zur Ketogruppe oxydiert werden. Vorzugsweise wird diese Oxydation mit Chromtrioxid in saurem Medium und in einem inerten organischen Lösungsmittel durchgeführt.

Die Verfahrensprodukte besitzen wertvolle pharmakologische Eigenschaften. Sie sind insbesondere lokal und topisch sehr stark antiphlogistisch wirksam und zeigen teilweise ein überraschend gutes Verhältnis von lokaler zu systemischer antiinflammtorischer Wirkung, wie aus pharmakologischen Standardtests hergeleitet werden kann.

Demgemäß ist Gegenstand der Erfindung auch ein Mittel zur Behandlung entzündlicher Dermatosen

bestehend aus oder enthaltend eine Verbindung der Formel I.

Die Verfahrensprodukte können in der Veterinär- und Humantherapie zur Behandlung von entzündlichen Dermatosen verschiedenster Genese in Form von Suspensionen, Salben, Cremes, Sprays usw. Verwendung finden.

Dabei ist als besonders vorteilhaft für die lokale und topische Therapieform herauszuheben, daß die Verfahrensprodukte aufgrund ihres sehr günstigen Verhältnisses von lokaler zu systemischer antiphlogistischer Wirkung auch bei hochdosierter und langanhaltender Therapie praktisch nur geringfügige systemische Nebenwirkungen hervorrufen können. Bei äußerlicher Behandlung werden Salben, Cremes, Suspensionen usw. mit einer Konzentration von 0,01 bis 2 Gew.-% verwendet. Insbesondere zeigen die Verfahrensprodukte in pharmakologischen Tests einen zum Teil besseren Split (Verhältnis) von lokaler/systemischer antiinflammatorischer Wirkung als entsprechende Präparate mit linearer 17-Alkylcarbonatgruppe, zu denen auch das in der europäischen Patentschrift Nr. 742 beschriebene Prednicarbat zählt. Weiterhin zeigen die Verfahrensprodukte teilweise auch eine stärkere lokale antiphlogistische Wirksamkeit als ihre bekannten Cortocoid-Analogen mit im Alkylrest der 17-Alkylcarbonatgruppen linear angeordneten Alkylanteil.
Darüberhinaus versprechen Corticoid-17-alkylcarbonat-21-derivate mit im Alkylanteil verzweigter 17-Alkylcarbonatgruppe gegenüber analogen Corticoid-17-alkylcarbonat-21-derivaten mit linear angeordneter 17-Alkylcarbonatseitenkette eine noch geringere Atrophogenität, was ein weiterer Vorteil für eine dermatotherapeutische Behandlung wäre.

Pharmakologischer Versuchsteil

So zeigte z.B. Prednisolon-17-iso-propylcarbonat-21-acetat (Beispiel 4, vierte Verbindung) = Verbindung I [Schmpkt. 126 °-128 °C; DC: $R_F \cong 0{,}7$], im Vergleich zu dem isomeren bekannten (EP 742) Prednisolon-17-n-propylcarbonat-21-acetat = Verbindung II, einmal eine etwa 3 mal stärkere lokale antiphlogistische Wirkung und zum anderen auch einen deutlich besseren Split von lokalter/systemischer antiphlogistischer Wirkung, wie aus den unten angeführten pharmakologischen Tests hervorgeht:

1. Lokale antiphlogistische Wirkung im Crotonöl-Ohrödem an Ratten nach epikutaner Applikation

Wir verwendeten die Rattenohr-Methode von Tonelli et al.: Männliche Wistar-Ratten aus eigener Zucht im Gewicht um 50 g wurden am rechten Ohr mit dem Irritans bzw. Testsubstanz enthaltenden Irritans epikutan (20 µl innen bzw. außen) behandelt. Das linke Ohr blieb unbehandelt. Zur Entzündungsauslösung diente wiederum Crotonöl (C), das in folgenden Lösungsmittelgemisch vorlag: C:Pyridin:Ethanol:Ether wie 4:20:10:66. Die zu prüfenden Kortikoide wurden hierin in den angegebenen Endkonzentrationen gelöst. Kontrollen erhielten nur das C-Lösungsmittelgemisch. 4 h nach epikutaner Behandlung wurden die Tiere mit Ether narkotisiert. Aus dem rechten (behandelten) und dem linken (unbehandelten) Ohr wurden 8 mm Durchmesser messende Scheiben ausgestanzt und sofort gewogen. Diese Differenz als Paramter für den Grad der Entzündung bei Kontrollen (mg, $\bar{x} \pm s$) wurde = 100 gesetzt. Die antiphlogistische Wirkung wird durch Angabe der ca. 50 %-igen Hemmdosis in mg/ml charakterisiert:

**Verbindung I:**

| | Behandlung mg/ml | $\bar{x}$ | ∓ s(mg) | Hemmung in % |
|---|---|---|---|---|
| Kontrolle | - | 12,0 | 3,8 | - |
| Verbdg. I | 0,30 | 3,0 | 2,4 | 75 % |
| Verbdg. II | 0,30 | 5,0 | 3,6 | 58 % |

**Hieraus ergibt sich als ca. 50 %-ige Hemmdosis:**

**Verbindung I = 0,1 mg/ml**

**Verbindung II = 0,3 mg/ml.**

2. Prüfung auf systemische antiphlogistische Wirkung im Test "Antiphlogistische Wirkung nach subcutaner Gabe: Carrageenan-Pfotenödem an Ratten".

Als Test für die akute systemische antiphlogistische Wirkung wurde das Carrageenan-Pfotenödem an

Ratten nach der von Winter et al. (1962) beschriebenen Methode gewählt. Männliche Sprague-Dawley-Ratten im Gewicht um 120 g erhielten die zu prüfenden Substanzen s.c. (0,2 ml/100 g) in Sesamöl gelöst. 30 min später wurde in die linke Hinterpfote 0,1 bis 0,5 % Carrageenan-Lösung injiziert. 6 h später wurde die Schwellungszunahme volumetrisch gemessen. Kontrollen erhielten nur Sesamöl.

Die Pfotenvolumina sind in ml, $\bar{x} \pm s$, angegeben. Die antiphlogistische Wirkung wird auch hier durch Angabe der ca. 50 %igen Hemmdosis in mg/kg charakterisiert.

| | Dosis in mg/kg | Ausgangswert (ml) MW ± S | Vol. Zunahme (ml) nach 6 h MW ± S |
|---|---|---|---|
| Kontrolle | - | 1,26 ± 0,18 | 0,53 ± 0,14 |
| Verbdg. I | 0,3 | 1,28 ± 0,07 | 0,49 ± 0,23 |
| | 3,0 | 1,34 ± 0,11 | 0,55 ± 0,10 |
| Verbdg. II | 0,3 | 1,25 ± 0,13 | 0,60 ± 0,11 |
| | 3,0 | 13,2 ± 0,10 | 0,13 ± 0,11 |

Hieraus ergibt sich als ca. 50 %ige Hemmdosis:

Verbindung I: > 3,0 mg/kg

Verbindung II: ~ 0,5 mg/kg

Aufgrund der oben erhaltenen Ergebnisse aus Test 1 und 2, ergibt sich für das Präperatepaar I und II beim gleichen Tier Ratte einmal eine etwa 3 mal stärkere lokale antiphlogistische Wirkung von I gegenüber II und zum anderen eine Verringerung der systemischen Wirkung von I gegenüber II um den Faktor >6. Hierdurch ist der um eine Größenordnung günstigere Split von Prednisolon-17-iso-propylcarbonat-21-acetat = I, gegenüber Prednisolon-17-n-propylcarbonat21-acetat = II eindeutig dokumentiert.

Darüberhinaus können die erfindungsgemäßen Verfahrensprodukte mit diversen gut hautverträglichen lokal wirksamen Antibiotika, z.B. vom Typ des Gentamycins, Neomycins, Erythromycins, Tetracyclins oder der Fusidinsäure und anderen, in galenischen Formulierungen kombiniert werden. Derartige Kombinationen aus den Verfahrensprodukten und den lokal wirksamen Antibiotika können zur Behandlung von primären bakteriellen oder bakteriell superinfizierten entzündlichen Dermatosen verwendet werden.

## Beispiele

Zu den im folgenden aufgeführten Beispielen sind die nachstehenden allgemeinen Bemerkungen zu machen:
Die Schmelzpunkte werden im Apparat nach Tottoli (Fa. Büchi) bestimmt und sind nicht korrigiert.

Die IR-Spektren (in KBr) werden mit dem Gitterspektrophotometer Perkin-Elmer 521 aufgenommen. Es werden jeweils nur die charakteristischen Banden angeführt. Die Aufnahme der UV-Spektren (in Methanol) erfolgte mit dem Spektralphotometer Beckmann DK 1 A. Die massenspektroskopischen Untersuchungen (MS) werden vorwiegend mit dem Gerät MS 9 (Fa. AEI) durchgeführt. Angabe der MS-Spektren (Molgewichtspeak) überwiegend in:
MS = m/z = ..... ($M+H^+$) (Messung mit Reinistopen). In der Regel wurden FAB-MS-Spektren gemessen.

Für die Dünnschichtchromatographie (DC) dienten Fertigplatten Kieselgel $F_{254}$ (Fa. Merck).
Wenn nicht anders angegeben, wurde als Laufmittel Methylenchlorid : Methanol = 19 : 1 benutzt (Laufstrecke 7 cm). Es wurde jeweils zweimal entwickelt. Die Flecken wurden entweder mit einer UV-Lampe bei 254 nm detektiert oder durch Besprühen mit 10 %-iger methanolischer Schwefelsäure sowie durch Erhitzen auf 100 °C sichtbar gemacht. Die $R_f$-Werte sind immer nur relativ zu verstehen. Zur Säulenschromatographie wurde 15 Kieselgel 60, Korngröße 0,063 - 0,2 mm (Fa. Merck) verwendet.

**Beispiel 1**

a.1.) Eine Lösung 1,2 g Prednisolon-17,21-di-iso-propyl-orthocarbonat (DC:$R_F$ etwa 0,65) in 18 ml Eisessig und 0,18 ml Wasser wird 5 Stunden bei 22 °C stehen gelassen. Eine DC-Überprüfung ergab, daß nach dieser Zeit eine optimale Menge an dem gewünschten Prednisolon-17-isopropylcarbonat vorhanden war.
Man gießt das Reaktionsgemisch in 0,5 l Wasser ein, das mit Ammoniak-Lösung auf pH = 5 gebracht worden war, wobei ein kristalliner Niederschlag ausfiel. Nach dem Abfiltrieren, Waschen mit Wasser und Trocknen erhält man nach dem Digerieren 0,7 g Prednisolon-17-isopropylcarbonat vom Schmp. 128 °C (Tottoli). Das zurückgebliebene wässerige Filtrat wird mit Methylenchlorid extrahiert. Nach dem Abdestillieren des Lösungsmittels bleibt ein schaumiger Rückstand zurück, der aus Diisopropylether zur Kristallisation gebracht wird und weitere 0,3 g Prednisolon-isopropylcarbonat von Schmp. 126° ergibt. Beide Präparationen werden vereinigt und aus Ethanol umkristallisiert.
Schmp. 131 °C (Tottoli)
Massenspektrum: MS: m/z = 447 ($M+H^+$)
DC: $R_f \cong 0,45$
($CH_2Cl_2$:$CH_3OH$ = 19:1)
Charakter. IR-Banden: 3450, 2940, 2870, 1740, 1720, 1270 $cm^{-1}$.
a.2.) Zum gleichen Reaktionsprodukt konnte man, wenn man wie folgt verfährt:
Eine Lösung bzw. Suspension von 24 g Prednisolon-17,21-di-isopropyl-orthocarbonat in 120 ml Eisessig und 50 g Ammoniumacetat wird 2 Std. bei 22 °C gerührt und nach DC-Kontrolle (siehe a.1.)) in 3 l mit NaCl gesättigtes Wasser eingerührt. Das ausgefallene Öl wird nach Abdekantieren der wässrigen Phase über ein Faltenfilter angereichert und mit säurefreiem Methylenchlorid extrahiert. Die organische Phase wird mit Wasser gewaschen und mit Natriumsulfat getrocknet. Nach dem Abdestillieren des Lösungsmittels erhält man 18,5 g Prednisolon-17-iso-propylcarbonat als Öl bzw. Schaum, das laut DC ($R_f$ etwa 0,45) einheitlich ist und nach dem Trocknen im Hochvakuum ohne Weiterbehandlung in die Folgereaktionen eingesetzt werden kann.
Zur kristallisierten Darstellung werden 5 g aus Ethanol/Di-Diethylether kristallisiert. Man erhält das gleiche Reaktionsprodukt wie unter a.1.) beschrieben mit dem Schmp. 131 °C und DC ($R_F$ = 0,45)
b.) In gleicher Weise, wie in Beispiel 1) a.2.) beschrieben, werden 35 g Prednisolon-17,21-di-isobutylorthocarbonat in 175 ml Eisessig und mit 73 g Ammoniumacetat umgesetzt und aufgearbeitet. Man erhält 28,4 g Prednisolon-17-isobutyl-carbonat als Schaum.
DC = $R_F$ etwa 0,5
MS: m/z = 461 ($M+H^+$)
c.) In gleicher Weise, wie in Beispiel 1.) a.2.) beschrieben, werden 43 g Prednisolon-17,21-di-tert.-butylmethylorthocarbonat in 201 ml Eisessig und mit 89 g Ammoniumacetat umgesetzt und aufgearbeitet. Das ölige Reaktionsprodukt (34 g) wird an Kieselgel 35-70 (Säulenmaße 5,5 x 41 cm) chromatographisch gereinigt. Nach Durchsatz von 3 l Methylenchlorid/Methanol -89:2 (Nebenprodukte laut DC), werden 1,5 l Eluationsmittel Methylenchlorid/Methanol = 96:4 durchgesetzt. Nach Abdestillieren der Lösungsmittel erhält man das gewünschte Prednisolon-17-tert.-butylmethylcarbonat als helles Öl.
DC etwa 0,5
MS: m/z = 475 ($M+H^+$)
d.) In ähnlicher Weise, wie in Beispiel 1) a.2.) beschrieben, werden 21 g Prednisolon-17,21-di-methoxyethylorthocarbonat in 105 ml Eisessig und 44 g Ammoniumacetat 4 Std. bei 35-40 °C gerührt und aufgearbeitet. Das erhaltene ölige Reaktionsprodukt (13,5 g) wird aus Diethylether zur Kristallisation gebracht und ergibt 8,2 g Prednisolon-17-methoxyethylcarbonat vom Schmp. 131 °C
MS: m/z = 463 ($M+H^+$)

**Beispiel 2**

a.) Zu einer Lösung von 6 g Prednisolon-17-isobutylcarbonat in 55 ml Pyridin werden 35 ml Essigsäureanhydrid zugegeben. Nach 4 Std. Rühren bei 20 °C, wird in 1,8 l halbgesättigte wässrige Kochsalzlösung eingerührt, wobei ein Öl ausfällt. Die wässrige Phase wird abdekantiert, das Öl mit Methylenchlorid aufgenommen die organische Phase mit Wasser gewaschen und mit Natriumsulfat getrocknet. Nach dem Abdestillieren des Lösungsmittels hinterbleibt ein Öl, das aus Diethylether zur Kristallisation gebracht wird. Man erhält 3,5 g kristallisiertes Prednisolon-17-isobutylcarbonat-21-acetat vom Schmp. 185 °C,
MS: m/z 503 ($M+H^+$)

b.) Zu einer Lösung 6,85 g Prednisolon-17-isobutylcarbonat in 68 ml absol. Pyridin werden unter Rühren und bei 0 °C innerhalb 60 min. 2 g reinstes Propionsäurechlorid in 2 ml Dioxan zugetropft. Nach 30 Minuten Rühren bei 0 °C und 3 Stunden Rühren bei 20 °C wird das Reaktionsgemisch in 1,8 l Wasser, das 100 g NaCl enthält, eingegossen. Der ölige Niederschlag wird abfiltriert, gut mit Wasser gewaschen und im Hochvakuum getrocknet. Man erhält 6,3 g Schaum, der im DC neben dem Hauptfleck bei $R_F$ = 0,7 noch schwache Nebenflecke bei $R_F$ etwa 0,5-0,55 aufweist.
Zur Reindarstellung wird an 150 g Kieselgel (Korngröße 0,063-0,200 mm (Merck AG); Säule 28 x 5 cm) mit 1,7 1 Methylenchlorid und 800 ml Methylenchlorid/Methanol = 995 : 5 chromatographiert. Nach dem Abdestillieren der Eluationsmittel erhält man aus Diethylether 3,8 g kristallisiertes Prednisolon-17-isobutylcarbonat-21-n-propionat vom Schmp. 136 °C
MS; m/z = 571 $(M+H^+)$
DC:$R_F \cong 0,7$

**Beispiel 3**

In gleicher Weise, wie in Beispiel 1 a.1.) oder a.2.) beschrieben, werden aus den Corticosteroid-17,21-ortho-alkylcarbonaten der Formel V die in Tabelle 1 aufgeführten Corticosteroid-17-alkylcarbonate mit freier 21-Hydroxylgruppe (R(1) = OH) der Formel I erhalten:

**Tabelle 1**

| Basis-Costicosteroid | Verfahrens-Variante (Beispiel 1) | A | Y | Z | R(2) | R(3) | Massenspektrum (m/z) M+H$^+$ | DC: $R_f$ |
|---|---|---|---|---|---|---|---|---|
| Dexamethason | a.2.) | CHOH | F | H | $-CH(CH_3)_2$ | $CH_3$ | 479 | 0,55 |
| " | " | " | " | " | $-CH_2CH(CH_3)_2$ | " | 493 | 0,55 |
| " | " | " | " | " | $-CH_2C(CH_3)_3$ | " | 507 | 0,55 |
| " | " | " | " | " | $-CH_2CH_2OCH_3$ | " | 495 | 0,5 |
| 6α-Methyl-prednisolon | a.2.) | CHOH | H | $CH_3$ | $-CH(CH_3)_2$ | H | 461 | |
| " | " | " | " | " | $-CH_2CH(CH_3)_2$ | " | 475,4 | 0,5 |
| " | " | " | " | " | $-CH_2C(CH_3)_3$ | " | 489 | 0,5 |
| " | " | " | " | " | $-CH_2CH_2OCH_3$ | " | 477 | |
| 6α-Fluor-dexamethason | a.2.) | CHOH | F | F | $-CH(CH_3)_2$ | $CH_3$ | 497 | 0,5 |
| " | " | " | " | " | $-CH_2CH(CH_3)_2$ | " | 511 | |
| 6α-Fluor-dexamethason | " | " | " | " | $-CH_2C(CH_3)_3$ | " | 525 | |
| " | " | " | " | " | $-CH_2CH_2OCH_3$ | " | 513 | |
| Cortisol (1,2-Dihydro) | a.2.) | CHOH | H | H | $-CH(CH_3)_2$ | H | 449 | |
| " " | " | " | " | " | $-CH_2CH(CH_3)_3$ | H | 463 | 0,5 |
| Cortison (1,2-Dihydro) | a.2.) | C=O | H | H | $-CH(CH_3)_2$ | H | 447 | |
| " " | a.1.) | " | " | " | $-CH_2CH(CH_3)_2$ | H | 461 | |
| Cortisol | " | " | " | " | $-CH_2C(CH_3)_3$ | " | 477 | |
| " | " | " | " | " | $-CH_2CH_2OCH_3$ | " | 465 | |

Forsetzung von Tabelle 1

| Basis-Costicosteroid | Verfahrens-Variante (Beispiel 1) | A | Y | Z | R(2) | R(3) | Massenspektrum (m/z) $M+H^+$ | DC: $R_f$ |
|---|---|---|---|---|---|---|---|---|
| Prednison | a.2.) | C=O | H | H | $-CH(CH_3)_2$ | H | 445 | 0,5 |
| " | " | " | " | " | $-CH_2CH(CH_3)_2$ | H | 459 | |
| " | " | " | " | " | $-CH_2C(CH_3)_3$ | " | 473 | |
| " | " | " | " | " | $-CH_2CH_2OCH_3$ | " | 461 | |
| 6α-Fluorprednisolon | a.2.) | CHOH | H | F | $-CH_2CH(CH_3)_2$ | H | 479 | 0,55 |
| 6α,16α-Dimethyl-prednisolon | a.2.) | CHOH | H | $CH_3$ | $-CH_2CH(CH_3)_2$ | $CH_3$ | 489 | |
| Reichsteins Substanz S | a.2.) | $-CH_2-$ | H | H | $-CH_2CH(CH_3)_2$ | H | 447 | 0,6 |
| " | " | " | " | " | $-CH(CH_3)_2$ | H | 433 | |

## Beispiel 4

In gleicher Weise werden aus Prednisolon-17-isopropyl-carbonat, aus Prednisolon-17-tert.-butylmethyl-carbonat, aus Prednisolon-17-methoxyethylcarbonat,

wenn man 1.) nach Beispiel 2, b.) verfährt, erhalten:
Prednisolon-17-isopropylcarbonat-21-propionat
MS = m/z = 503 ($M+H^+$)
Prednisolon-17-tert.-butylmethylcarbonat-21-propionat
MS = m/z 531 ($M+H^+$)
Prednisolon-17-methoxyethylcarbonat-21-propionat
MS = m/z = 519 ($M+H^+$)
wenn man 2.) nach Beispiel 2, a.) verfährt, erhalten:
Prednisolon-17-iso-propylcarbonat-21-acetat
MS = m/z = 489 ($M+H^+$),
Prednisolon-17-tert.-butylmethylcarbonat-21-acetat
MS = m/z = 505 ($M+H^+$),
Prednisolon-17-methoxyethylcarbonat-21-acetat
Ms = m/z = 505 ($M+H^+$)

**Beispiel 5**

In gleicher oder ähnlicher Weise wie in Beispiel 2.a.) und 2.b.) beschrieben, werden folgende Corticosteroid-17-alkylcarbonat-21-carbonsäureester (R(1) = $OCO(CH_2)_n$-R(4)), -21-Kohlensäureester (R(1) = $OCO_2(CH_2)_n$-R(4)) oder 21- Alkyl- bzw. Arylsulfonsäureester (R(1) = $OSO_2$R(5)) der Formel I gemäß Tabellen 2-7 hergestellt bzw. erhalten, wenn bei Beispiel 2.a.) anstatt Essigsäureanhydrid die analogen Carbonsäureanhydride ($CO[CO(CH_2)_nR(4)]_2$) oder anstelle von Propionsäurechlorid bei Beispiel 2.b.) die entsprechenden Carbonsäurechloride (Cl-CO-$(CH_2)_n$R(4)) oder Kohlensäurechloride bzw. Chlorameisensäureester (Cl-CO-O-$(CH_2)_n$R(4)) oder Alkyl- bzw. Arylsulfonsäurechloride (Cl-$SO_2$-R(5)) eingesetzt werden.

Die Bedeutungen von A, Y, Z, R(3), R(2), R(1), R(4), R(5) sind eingangs zu Formel I angegeben.

**Anmerkung**

Bei den entsprechenden Reaktionen mit Alkyl- oder Arylsulfonsäurechloriden wird in vorteilhafter Weise absolutes Aceton zum Reaktionsgemisch hinzugefügt, wobei das Verhältnis Aceton/Pyridin etwa 10:4 ist.

Bei den Reaktionen mit Carbonsäurechloriden wird in vorteilhafter Weise oft absolutes Dioxan zum Reaktionsgemisch gegeben, z.B. bei Cyclopropylcarbonsäurechlorid, wobei das Verhältnis Dioxan/Pyridin etwa 1:1 ist, und zur Reaktionsbeschleunigung wird das Reaktionsgemisch oft, insbesondere bei Cyclopropylcarbonsäurechlorid, auf etwa 60 °C erwärmt (DC-Verfolgung der Reaktionsverläufe).

Die Charakterisierung der Reaktionsprodukte kann durch Dünnschichtchromatographie (DC) erfolgen; hierbei haben die Reaktionsprodukte $R_F$-Werte von etwa 0,65-0,75. In der Regel werden die Reaktionsprodukte durch Massenspektren mit MS = m/z = .... ($M+H^+$) charakterisiert (In der Regel FAB-Spektren). Die $M+H^+$-Werte wurden jeweils aufgerundet. Auch IR-, $^1$H-NMR- und UV-Spektren können zur Charakterisierung herangezogen werden.

**Tabelle 2a**

Basis-Corticoid = Prednisolon (Pred.)

| Reagenz: (Anhydrid oder Chlorid) | Basis-Corticoid | Verf.-Variante Beispiel 2 | A | Y | Z | R(3) | R(2) | R(1) | MS (m/z) M+H+ |
|---|---|---|---|---|---|---|---|---|---|
| $CH_3(CH_2)_2COCl$ | Pred. | 2.b.) | CHOH | H | H | H | $-CH(CH_3)_2$ | $OCO(CH_2)_2CH_3$ | 517 |
| $CH_3(CH_2)_3COCl$ | " | " | " | " | " | " | " | $OCO(CH_2)_3CH_3$ | 531 |
| $CH_3(CH_2)_4COCl$ | " | " | " | " | " | " | " | $OCO(CH_2)_4CH_3$ | 545 |
| $(CH_3)_2CHCOCl$ | " | " | " | " | " | " | " | $OCOCH(CH_3)_2$ | 517 |
| $(CH_3)_3CCOCl$ | " | " | " | " | " | " | " | $OCOC(CH_3)_3$ | 531 |
| 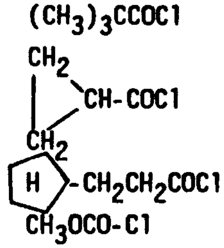 | " | " | " | " | " | " | " | 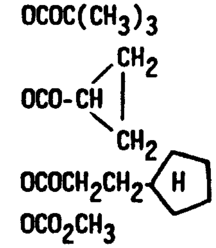 | 515 |
| $-CH_2CH_2COCl$ | " | " | " | " | " | " | " | $OCOCH_2CH_2$- | 571 |
| $CH_3OCO-Cl$ | " | " | " | " | " | " | " | $OCO_2CH_3$ | 505 |
| $C_2H_5OCO-Cl$ | " | " | " | " | " | " | " | $OCO_2C_2H_5$ | 519 |
| $C_3H_7-OCO-Cl$ | " | " | " | " | " | " | " | $OCO_2C_3H_7$ | 533 |
| $CH_3SO_2-Cl$ | " | " | " | " | " | " | " | $OSO_2CH_3$ | 529 |
| $C_6H_5SO_2-Cl$ | " | " | " | " | " | " | " | $OSO_2C_6H_5$ | 587 |
| $p-Cl-C_6H_4-SO_2-Cl$ | " | " | " | " | " | " | " | $OSO_2-C_6H_4-p-Cl$ | 622 |
| $p-CH_3-C_6H_4-SO_2-Cl$ (p-Tosylchlorid) | " | " | " | " | " | " | " | $OSO_2-C_6H_4-p-CH_3$ | 601 |

Tabelle 2b

Basis-Corticoid = Prednisolon (Pred.)

| Reagenz: (Anhydrid oder Chlorid) | Basis-Corticoid | Verf.-Variante Beispiel 2 | A | Y | Z | R(3) | R(2) | R(1) | MS (m/z) $M+H^+$ |
|---|---|---|---|---|---|---|---|---|---|
| $CH_3(CH_2)_2COCl$ | Pred. | 2.b.) | CHOH | H | H | H | $-CH_2CH(CH_3)_2$ | $OCO(CH_2)_2CH_3$ | 531 |
| $CH_3(CH_2)_3COCl$ | " | " | " | " | " | " | " | $OCO(CH_2)_3CH_3$ | 545 |
| $CH_3(CH_2)_4COCl$ | " | " | " | " | " | " | " | $OCO(CH_2)_4CH_3$ | 559 |
| $(CH_3)_2CHCOCl$ | " | " | " | " | " | " | " | $OCOCH(CH_3)_2$ | 531 |
| $(CH_3)_3CCOCl$ | " | " | " | " | " | " | " | $OCOC(CH_3)_3$ | 545 |
| 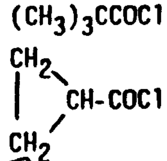 | " | " | " | " | " | " | " | 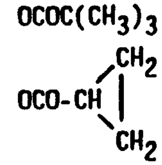 | 529 |
| 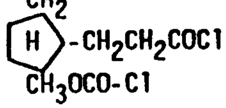 | " | " | " | " | " | " | " | 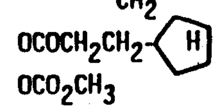 | 585 |
| $CH_3OCO-Cl$ | " | " | " | " | " | " | " | $OCO_2CH_3$ | 519 |
| $C_2H_5OCO-Cl$ | " | " | " | " | " | " | " | $OCO_2C_2H_5$ | 533 |
| $C_3H_7-OCO-Cl$ | " | " | " | " | " | " | " | $OCO_2C_3H_7$ | 547 |
| $CH_3SO_2-Cl$ | " | " | " | " | " | " | " | $OSO_2CH_3$ | 539 |
| $C_6H_5SO_2-Cl$ | " | " | " | " | " | " | " | $OSO_2C_6H_5$ | 601 |
| $p-Cl-C_6H_4-SO_2-Cl$ | " | " | " | " | " | " | " | $OSO_2-C_6H_4-p-Cl$ | 636 |
| $p-CH_3-C_6H_4-SO_2-Cl$ (p-Tosylchlorid) | " | " | " | " | " | " | " | $OSO_2-C_6H_4-p-CH_3$ | 615 |

**Tabelle 2c**

Basis-Corticoid = Prednisolon (Pred.)

| Reagenz: (Anhydrid oder Chlorid) | Basis-Corticoid | Verf.-Variante Beispiel 2 | A | Y | Z | R(3) | R(2) | R(1) | MS (m/z) $M+H^+$ |
|---|---|---|---|---|---|---|---|---|---|
| $CH_3(CH_2)_2COCl$ | Pred. | 2.b.) | CHOH | H | H | H | $-CH_2C(CH_3)_3$ | $OCO(CH_2)_2CH_3$ | 545 |
| $CH_3(CH_2)_3COCl$ | " | " | " | " | " | " | " | $OCO(CH_2)_3CH_3$ | 559 |
| $CH_3(CH_2)_4COCl$ | " | " | " | " | " | " | " | $OCO(CH_2)_4CH_3$ | 573 |
| $(CH_3)_2CHCOCl$ | " | " | " | " | " | " | " | $OCOCH(CH_3)_2$ | 545 |
| $(CH_3)_3CCOCl$ | " | " | " | " | " | " | " | $OCOC(CH_3)_3$ | 559 |
| 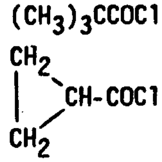 | " | " | " | " | " | " | " | 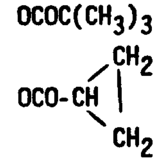 | 543 |
| $C_2H_5OCO-Cl$ | " | " | " | " | " | " | " | $OCO_2C_2H_5$ | 547 |
| $CH_3SO_2-Cl$ | " | " | " | " | " | " | " | $OSO_2CH_3$ | 553 |
| $p-Cl-C_6H_4-SO_2-Cl$ | " | " | " | " | " | " | " | $OSO_2-C_6H_4-p-Cl$ | 650 |

**Tabelle 2d**

Basis-Corticoid = Prednisolon (Pred.)

| Reagenz: (Anhydrid oder Chlorid) | Basis-Corticoid | Verf.-Variante Beispiel 2 | A | Y | Z | R(3) | R(2) | R(1) | MS (m/z) $M+H^+$ |
|---|---|---|---|---|---|---|---|---|---|
| $CH_3(CH_2)_2COCl$ | Pred. | 2.b.) | CHOH | H | H | H | $-CH_2CH_2OCH_3$ | $OCO(CH_2)_2CH_3$ | 533 |
| $CH_3(CH_2)_3COCl$ | " | " | " | " | " | " | " | $OCO(CH_2)_3CH_3$ | 547 |
| $(CH_3)_2CHCOCl$ | " | " | " | " | " | " | " | $OCOCH(CH_3)_2$ | 533 |
| 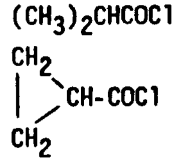 | " | " | " | " | " | " | " | 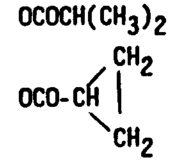 | 531 |
| $C_2H_5OCO-Cl$ | " | " | " | " | " | " | " | $OCO_2C_2H_5$ | 535 |
| $CH_3SO_2-Cl$ | " | " | " | " | " | " | " | $OSO_2CH_3$ | 545 |
| $p-Cl-C_6H_4-SO_2-Cl$ | " | " | " | " | " | " | " | $OSO_2-C_6H_4-p-Cl$ | 638 |

**Tabelle 3a**

Basis-Corticoid = Dexamethason (Dex.)

| Reagenz: (Anhydrid oder Chlorid) | Basis-Corticoid | Verf.-Variante Beispiel 2 | A | Y | Z | R(3) | R(2) | R(1) | MS (m/z) $m+H^+$ |
|---|---|---|---|---|---|---|---|---|---|
| $(CH_3CO)_2O$ | Dex. | 2.a.) | CHOH | F | H | $CH_3$ | $-CH(CH_3)_2$ | $OCOCH_3$ | 521 |
| $CH_3CH_2COCl$ | " | 2.b.) | " | " | " | " | " | $OCOC_2H_5$ | 535 |
| $CH_3(CH_2)_2COCl$ | " | " | " | " | " | " | " | $OCO(CH_2)_2CH_3$ | 549 |
| $CH_3(CH_2)_3COCl$ | " | " | " | " | " | " | " | $OCO(CH_2)_3CH_3$ | 563 |
| $CH_3(CH_2)_4COCl$ | " | " | " | " | " | " | " | $OCO(CH_2)_4CH_3$ | 577 |
| $(CH_3)_2CHCOCl$ | " | " | " | " | " | " | " | $OCOCH(CH_3)_2$ | 549 |
| $(CH_3)_3CCOCl$ | " | " | " | " | " | " | " | $OCOC(CH_3)_3$ | 563 |
| 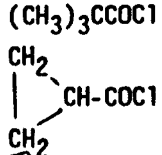 | " | " | " | " | " | " | " | 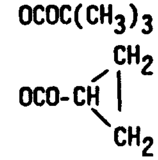 | 547 |
| 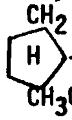$-CH_2CH_2COCl$ | " | " | " | " | " | " | " | $OCOCH_2CH_2$- H | 603 |
| $CH_3OCO-Cl$ | " | " | " | " | " | " | " | $OCO_2CH_3$ | 537 |
| $C_2H_5OCO-Cl$ | " | " | " | " | " | " | " | $OCO_2C_2H_5$ | 551 |
| $C_3H_7-OCO-Cl$ | " | " | " | " | " | " | " | $OCO_2C_3H_7$ | 565 |
| $CH_3SO_2-Cl$ | " | " | " | " | " | " | " | $OSO_2CH_3$ | 561 |
| $p-Cl-C_6H_4-SO_2-Cl$ | " | " | " | " | " | " | " | $OSO_2-C_6H_4-p-Cl$ | 654 |
| $p-CH_3-C_6H_4-SO_2-Cl$ (p-Tosylchlorid) | " | " | " | " | " | " | " | $OSO_2-C_6H_4-p-CH_3$ | 633 |

**Tabelle 3b**

Basis-Corticoid = Dexamethason (Dex.)

| Reagenz: (Anhydrid oder Chlorid) | Basis-Corticoid | Verf.-Variante Beispiel 2 | A | Y | Z | R(3) | R(2) | R(1) | MS (m/z) $M+H^+$ |
|---|---|---|---|---|---|---|---|---|---|
| $(CH_3CO)_2O$ | Dex. | 2.a.) | CHOH | F | H | $CH_3$ | $-CH_2CH(CH_3)_2$ | $OCOCH_3$ | 535 |
| $CH_3CH_2COCl$ | " | 2.b.) | " | " | " | " | " | $OCOC_2H_5$ | 549 |
| $CH_3(CH_2)_2COCl$ | " | " | " | " | " | " | " | $OCO(CH_2)_2CH_3$ | 563 |
| $CH_3(CH_2)_3COCl$ | " | " | " | " | " | " | " | $OCO(CH_2)_3CH_3$ | 577 |
| $CH_3(CH_2)_4COCl$ | " | " | " | " | " | " | " | $OCO(CH_2)_4CH_3$ | 591 |
| $(CH_3)_2CHCOCl$ | " | " | " | " | " | " | " | $OCOCH(CH_3)_2$ | 563 |
| $(CH_3)_3CCOCl$ | " | " | " | " | " | " | " | $OCOC(CH_3)_3$ | 577 |
| 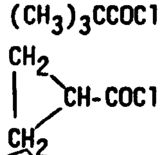 | " | " | " | " | " | " | " | 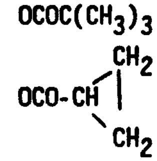 | 561 |
| 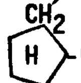 $-CH_2CH_2COCl$ | " | " | " | " | " | " | " | $OCOCH_2CH_2$- H | 617 |
| $CH_3OCO-Cl$ | " | " | " | " | " | " | " | $OCO_2CH_3$ | 551 |
| $C_2H_5OCO-Cl$ | " | " | " | " | " | " | " | $OCO_2C_2H_5$ | 565 |
| $C_3H_7-OCO-Cl$ | " | " | " | " | " | " | " | $OCO_2C_3H_7$ | 579 |
| $CH_3SO_2-Cl$ | " | " | " | " | " | " | " | $OSO_2CH_3$ | 571 |
| $C_6H_5-SO_2-Cl$ | " | " | " | " | " | " | " | $OSO_2C_6H_5$ | 633 |
| $p-Cl-C_6H_4-SO_2-Cl$ | " | " | " | " | " | " | " | $OSO_2-C_6H_4-p-Cl$ | 668 |
| $p-CH_3-C_6H_4-SO_2-Cl$ (p-Tosylchlorid) | " | " | " | " | " | " | " | $OSO_2-C_6H_4-p-CH_3$ | 647 |

**Tabelle 3c**

Basis-Corticoid = Dexamethason (Dex.)

| Reagenz: (Anhydrid oder Chlorid) | Basis-Corticoid | Verf.-Variante Beispiel 2 | A | Y | Z | R(3) | R(2) | R(1) | MS (m/z) $M+H^+$ |
|---|---|---|---|---|---|---|---|---|---|
| $(CH_3CO)_2O$ | Dex. | 2.a.) | CHOH | F | H | $CH_3$ | $-CH_2C(CH_3)_3$ | $OCOCH_3$ | 549 |
| $CH_3CH_2COCl$ | " | 2.b.) | " | " | " | " | " | $OCOC_2H_5$ | 563 |
| $CH_3(CH_2)_2COCl$ | " | " | " | " | " | " | " | $OCO(CH_2)_2CH_3$ | 577 |
| $(CH_3)_2CHCOCl$ | " | " | " | " | " | " | " | $OCOCH(CH_3)_2$ | 577 |
| 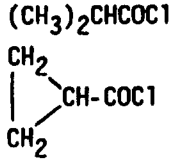 | " | " | " | " | " | " | " | 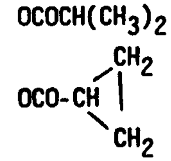 | 575 |
| $C_2H_5OCO-Cl$ | " | " | " | " | " | " | " | $OCO_2C_2H_5$ | 579 |
| $CH_3SO_2-Cl$ | " | " | " | " | " | " | " | $OSO_2CH_3$ | 585 |
| $p-Cl-C_6H_4-SO_2-Cl$ | " | " | " | " | " | " | " | $OSO_2-C_6H_4-p-Cl$ | 682 |

**Tabelle 3d**

Basis-Corticoid = Dexamethason (Dex.)

| Reagenz: (Anhydrid oder Chlorid) | Basis-Corticoid | Verf.-Variante Beispiel 2 | A | Y | Z | R(3) | R(2) | R(1) | MS (m/z) $M+H^+$ |
|---|---|---|---|---|---|---|---|---|---|
| $(CH_3CO)_2O$ | Dex. | 2.a.) | CHOH | F | H | $CH_3$ | $-CH_2CH_2OCH_3$ | $OCOCH_3$ | 537 |
| $CH_3CH_2COCl$ | " | 2.b.) | " | " | " | " | " | $OCOC_2H_5$ | 551 |
| $CH_3(CH_2)_2COCl$ | " | " | " | " | " | " | " | $OCO(CH_2)_2CH_3$ | 565 |
| $(CH_3)_2CHCOCl$ | " | " | " | " | " | " | " | $OCOCH(CH_3)_2$ | 565 |
| $(CH_3)_3CCOCl$ | " | " | " | " | " | " | " | $OCOC(CH_3)_3$ | 579 |
| 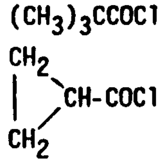 | " | " | " | " | " | " | " | 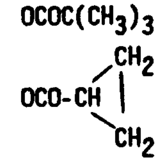 | 563 |
| $C_2H_5OCO-Cl$ | " | " | " | " | " | " | " | $OCO_2C_2H_5$ | 567 |
| $CH_3SO_2-Cl$ | " | " | " | " | " | " | " | $OSO_2CH_3$ | 577 |
| $p-Cl-C_6H_4-SO_2-Cl$ | " | " | " | " | " | " | " | $OSO_2-C_6H_4-p-Cl$ | 670 |

**Tabelle 4a**

Basis-Corticoid = 6α-Methylprednisolon (M-pred.)

| Reagenz: (Anhydrid oder Chlorid) | Basis-Corticoid | Verf.-Variante Beispiel 2 | A | Y | Z | R(3) | R(2) | R(1) | MS (m/z) $M+H^+$ |
|---|---|---|---|---|---|---|---|---|---|
| $(CH_3CO)_2O$ | M.pred. | 2.a.) | CHOH | H | $CH_3$ | H | $-CH(CH_3)_2$ | $OCOCH_3$ | 503 |
| $CH_3CH_2COCl$ | " | 2.b.) | " | " | " | " | " | $OCOC_2H_5$ | 517 |
| $CH_3(CH_2)_2COCl$ | " | " | " | " | " | " | " | $OCO(CH_2)_2CH_3$ | 531 |
| $(CH_3)_2CHCOCl$ | " | " | " | " | " | " | " | $OCOCH(CH_3)_2$ | 531 |
| $(CH_3)_3CCOCl$ | " | " | " | " | " | " | " | $OCOC(CH_3)_3$ | 545 |
| 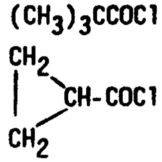 | " | " | " | " | " | " | " | 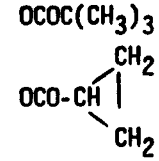 | 529 |
| $C_2H_5OCO-Cl$ | " | " | " | " | " | " | " | $OCO_2C_2H_5$ | 533 |
| $CH_3SO_2-Cl$ | " | " | " | " | " | " | " | $OSO_2CH_3$ | 543 |
| $p-Cl-C_6H_4-SO_2-Cl$ | " | " | " | " | " | " | " | $OSO_2-C_6H_4-p-Cl$ | 636 |

**Tabelle 4b**

Basis-Corticoid = 6α-Methyl-prednisolon (M.-pred.)

| Reagenz: (Anhydrid oder Chlorid) | Basis-Corticoid | Verf.-Variante Beispiel 2 | A | Y | Z | R(3) | R(2) | R(1) | MS (m/z) $M+H^+$ |
|---|---|---|---|---|---|---|---|---|---|
| $(CH_3CO)_2O$ | M.pred. | 2.a.) | CHOH | H | $CH_3$ | H | $-CH_2CH(CH_3)_2$ | $OCOCH_3$ | 517 |
| $CH_3CH_2COCl$ | " | 2.b.) | " | " | " | " | " | $OCOC_2H_5$ | 531 |
| $CH_3(CH_2)_2COCl$ | " | " | " | " | " | " | " | $OCO(CH_2)_2CH_3$ | 545 |
| $CH_3(CH_2)_3COCl$ | " | " | " | " | " | " | " | $OCO(CH_2)_3CH_3$ | 559 |
| $(CH_3)_2CHCOCl$ | " | " | " | " | " | " | " | $OCOCH(CH_3)_2$ | 545 |
| $(CH_3)_3CCOCl$ | " | " | " | " | " | " | " | $OCOC(CH_3)_3$ | 559 |
| 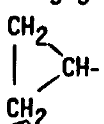-COCl | " | " | " | " | " | " | " | OCO-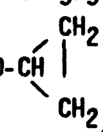 | 543 |
| 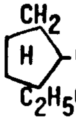$-CH_2CH_2COCl$ | " | " | " | " | " | " | " | $OCOCH_2CH_2$-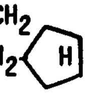 | 599 |
| $C_2H_5OCO-Cl$ | " | " | " | " | " | " | " | $OCO_2C_2H_5$ | 547 |
| $C_3H_7-OCO-Cl$ | " | " | " | " | " | " | " | $OCO_2C_3H_7$ | 561 |
| $CH_3SO_2-Cl$ | " | " | " | " | " | " | " | $OSO_2CH_3$ | 553 |
| $p-Cl-C_6H_4-SO_2-Cl$ | " | " | " | " | " | " | " | $OSO_2-C_6H_4-p-Cl$ | 650 |
| $p-CH_3-C_6H_4-SO_2-Cl$ (p-Tosylchlorid) | " | " | " | " | " | " | " | $OSO_2-C_6H_4-p-CH_3$ | 629 |

**Tabelle 4c**

Basis-Corticoid = 6α-Methylprednisolon (M.-pred.)

| Reagenz: (Anhydrid oder Chlorid) | Basis-Corticoid | Verf Variante Beispiel 2 | A | Y | Z | R(3) | R(2) | R(1) | MS (m/z) $M+H^+$ |
|---|---|---|---|---|---|---|---|---|---|
| $(CH_3CO)_2O$ | M.-pred | 2.a.) | CHOH | H | $CH_3$ | H | $-CH_2C(CH_3)_3$ | $OCOCH_3$ | 531 |
| $CH_3CH_2COCl$ | " | 2.b.) | " | " | " | " | " | $OCOC_2H_5$ | 545 |
| $(CH_3)_2CHCOCl$ | " | " | " | " | " | " | " | $OCOCH(CH_3)_2$ | 559 |
| $(CH_3)_3CCOCl$ | " | " | " | " | " | " | " | $OCOC(CH_3)_3$ | 573 |
| 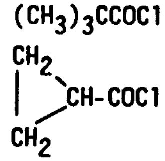 | " | " | " | " | " | " | " | 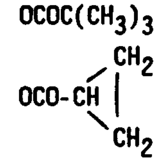 | 557 |
| $C_2H_5OCO-Cl$ | " | " | " | " | " | " | " | $OCO_2C_2H_5$ | 561 |
| $CH_3SO_2-Cl$ | " | " | " | " | " | " | " | $OSO_2CH_3$ | 567 |
| $p-Cl-C_6H_4-SO_2-Cl$ | " | " | " | " | " | " | " | $OSO_2-C_6H_4-p-Cl$ | 664 |

**Tabelle 4d**

Basis-Corticoid = 6α-Methylprednisolon (M.-pred.)

| Reagenz: (Anhydrid oder Chlorid) | Basis-Corticoid | Verf.-Variante Beispiel 2 | A | Y | Z | R(3) | R(2) | R(1) | MS (m/z) $M+H^+$ |
|---|---|---|---|---|---|---|---|---|---|
| $(CH_3CO)_2O$ | M.-pred | 2.a.) | CHOH | H | $CH_3$ | H | $CH_2CH_2OCH_3$ | $OCOCH_3$ | 519 |
| $CH_3CH_2COCl$ | " | 2.b.) | " | " | " | " | " | $OCOC_2H_5$ | 533 |
| $CH_3(CH_2)_2COCl$ | " | " | " | " | " | " | " | $OCO(CH_2)_2CH_3$ | 547 |
| 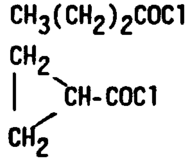 | " | " | " | " | " | " | " | 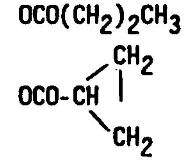 | 545 |
| $C_2H_5OCO-Cl$ | " | " | " | " | " | " | " | $OCO_2C_2H_5$ | 549 |
| $CH_3SO_2-Cl$ | " | " | " | " | " | " | " | $OSO_2CH_3$ | 559 |
| $p-Cl-C_6H_4-SO_2-Cl$ | " | " | " | " | " | " | " | $OSO_2-C_6H_4-p-Cl$ | 652 |

**Tabelle 5a**

Basis-Corticoid = 6α-Fluor-dexamethason (F-Dex.)

| Reagenz: (Anhydrid oder Chlorid) | Basis-Corticoid | Verf.-Variante Beispiel 2 | A | Y | Z | R(3) | R(2) | R(1) | MS (m/z) $M+H^+$ |
|---|---|---|---|---|---|---|---|---|---|
| $(CH_3CO)_2O$ | F-Dex. | 2.a.) | CHOH | F | F | $CH_3$ | $-CH(CH_3)_2$ | $OCOCH_3$ | 539 |
| $CH_3CH_2COCl$ | " | 2.b.) | " | " | " | " | " | $OCOC_2H_5$ | 553 |
| $CH_3(CH_2)_2COCl$ | " | " | " | " | " | " | " | $OCO(CH_2)_2CH_3$ | 567 |
| $CH_3(CH_2)_3COCl$ | " | " | " | " | " | " | " | $OCO(CH_2)_3CH_3$ | 581 |
| $(CH_3)_2CHCOCl$ | " | " | " | " | " | " | " | $OCOCH(CH_3)_2$ | 567 |
| 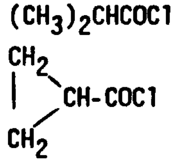 | " | " | " | " | " | " | " | 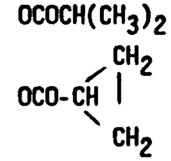 | 565 |
| $C_2H_5OCO-Cl$ | " | " | " | " | " | " | " | $OCO_2C_2H_5$ | 569 |
| $CH_3SO_2-Cl$ | " | " | " | " | " | " | " | $OSO_2CH_3$ | 579 |
| $p-Cl-C_6H_4-SO_2-Cl$ | " | " | " | " | " | " | " | $OSO_2-C_6H_4-p-Cl$ | 672 |

**Tabelle 5b**

Basis-Corticoid = 6α-Fluor-dexamethason (F-Dex.)

| Reagenz: (Anhydrid oder Chlorid) | Basis-Corticoid | Verf.-Variante Beispiel 2 | A | Y | Z | R(3) | R(2) | R(1) | MS (m/z) $M+H^+$ |
|---|---|---|---|---|---|---|---|---|---|
| $(CH_3CO)_2O$ | F-Dex. | 2.a.) | CHOH | F | F | $CH_3$ | $-CH_2CH(CH_3)_2$ | $OCOCH_3$ | 553 |
| $CH_3CH_2COCl$ | " | 2.b.) | " | " | " | " | " | $OCOC_2H_5$ | 567 |
| $CH_3(CH_2)_2COCl$ | " | " | " | " | " | " | " | $OCO(CH_2)_2CH_3$ | 581 |
| $CH_3(CH_2)_3COCl$ | " | " | " | " | " | " | " | $OCO(CH_2)_3CH_3$ | 595 |
| $(CH_3)_2CHCOCl$ | " | " | " | " | " | " | " | $OCOCH(CH_3)_2$ | 581 |
| 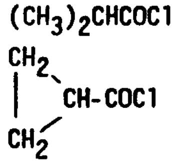 | " | " | " | " | " | " | " | 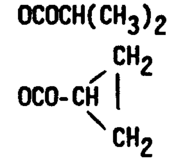 | 579 |
| $C_2H_5OCO-Cl$ | " | " | " | " | " | " | " | $OCO_2C_2H_5$ | 583 |
| $CH_3SO_2-Cl$ | " | " | " | " | " | " | " | $OSO_2CH_3$ | 589 |
| $p-Cl-C_6H_4-SO_2-Cl$ | " | " | " | " | " | " | " | $OSO_2-C_6H_4-p-Cl$ | 686 |

**Tabelle 5c**

Basis-Corticoid = 6α-Fluor-dexamethason (F-Dex.)

| Reagenz: (Anhydrid oder Chlorid) | Basis-Corticoid | Verf.-Variante Beispiel 2 | A | Y | Z | R(3) | R(2) | R(1) | MS (m/z) $M+H^+$ |
|---|---|---|---|---|---|---|---|---|---|
| $(CH_3CO)_2O$ | F-Dex. | 2.a.) | CHOH | F | F | $CH_3$ | $-CH_2C(CH_3)_3$ | $OCOCH_3$ | 567 |
| $CH_3CH_2COCl$ | " | 2.b.) | " | " | " | " | " | $OCOC_2H_5$ | 581 |
| $p-Cl-C_6H_4-SO_2-Cl$ | " | " | " | " | " | " | " | $OSO_2-C_6H_4-p-Cl$ | 700 |

**Tabelle 5d**

Basis-Corticoid = 6α-Fluor-dexamethason (F-Dex.)

| Reagenz: (Anhydrid oder Chlorid) | Basis-Corticoid | Verf.-Variante Beispiel 2 | A | Y | Z | R(3) | R(2) | R(1) | MS (m/z) $M+H^+$ |
|---|---|---|---|---|---|---|---|---|---|
| $(CH_3CO)_2O$ | F-Dex. | 2.a.) | CHOH | F | F | $CH_3$ | $CH_2CH_2OCH_3$ | $OCOCH_3$ | 555 |
| $CH_3CH_2COCl$ | " | 2.b.) | " | " | " | " | " | $OCOC_2H_5$ | 569 |
| $CH_3(CH_2)_2COCl$ | " | " | " | " | " | " | " | $OCO(CH_2)_2CH_3$ | 583 |
| 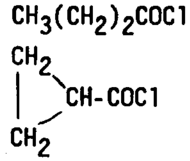 | " | " | " | " | " | " | " | 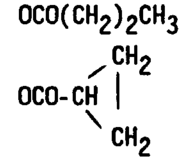 | 581 |
| $C_2H_5OCO-Cl$ | " | " | " | " | " | " | " | $OCO_2C_2H_5$ | 585 |
| $p-Cl-C_6H_4-SO_2-Cl$ | " | " | " | " | " | " | " | $OSO_2-C_6H_4-p-Cl$ | 688 |

**Tabelle 6a**

Basis-Corticoid = Cortisol (Cort.)
(in 1,2-Pos.: 1,2-Didhydro)

| Reagenz: . (Anhydrid oder Chlorid) | Basis-Corticoid | Verf.-Variante Beispiel 2 | A | Y | Z | R(3) | R(2) | R(1) | MS (m/z) $M+H^+$ |
|---|---|---|---|---|---|---|---|---|---|
| $(CH_3CO)_2O$ | Cort. | 2.a.) | CHOH | H | H | H | $-CH(CH_3)_2$ | $OCOCH_3$ | 491 |
| $CH_3CH_2COCl$ | " | 2.b.) | " | " | " | " | " | $OCOC_2H_5$ | 505 |
| $CH_3(CH_2)_2COCl$ | " | " | " | " | " | " | " | $OCO(CH_2)_2CH_3$ | 519 |
| $CH_3(CH_2)_3COCl$ | " | " | " | " | " | " | " | $OCO(CH_2)_3CH_3$ | 533 |
| $(CH_3)_2CHCOCl$ | " | " | " | " | " | " | " | $OCOCH(CH_3)_2$ | 519 |
| 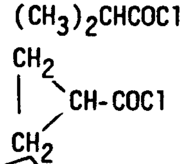 | " | " | " | " | " | " | " | 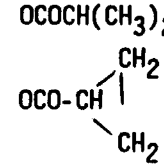 | 517 |
| 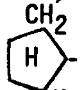$-CH_2CH_2COCl$ | " | " | " | " | " | " | " | $OCOCH_2CH_2-$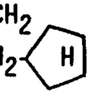 | 573 |
| $C_2H_5OCO-Cl$ | " | " | " | " | " | " | " | $OCO_2C_2H_5$ | 521 |
| $CH_3SO_2-Cl$ | " | " | " | " | " | " | " | $OSO_2CH_3$ | 531 |
| $p-Cl-C_6H_4-SO_2-Cl$ | " | " | " | " | " | " | " | $OSO_2-C_6H_4-p-Cl$ | 624 |

**Tabelle 6b**

Basis-Corticoid = Cortisol (Cort.)

| Reagenz: (Anhydrid oder Chlorid) | Basis-Corticoid | Verf.-Variante Beispiel 2 | A | Y | Z | R(3) | R(2) | R(1) | MS (m/z) $M+H^+$ |
|---|---|---|---|---|---|---|---|---|---|
| $(CH_3CO)_2O$ | Cort. | 2.a.) | CHOH | H | H | H | $-CH_2CH(CH_3)_2$ | $OCOCH_3$ | 505 |
| $CH_3CH_2COCl$ | " | 2.b.) | " | " | " | " | " | $OCOC_2H_5$ | 519 |
| $CH_3(CH_2)_2COCl$ | " | " | " | " | " | " | " | $OCO(CH_2)_2CH_3$ | 533 |
| $CH_3(CH_2)_3COCl$ | " | " | " | " | " | " | " | $OCO(CH_2)_3CH_3$ | 547 |
| $(CH_3)_2CHCOCl$ | " | " | " | " | " | " | " | $OCOCH(CH_3)_2$ | 533 |
| 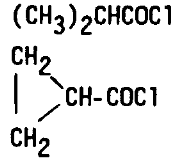 | " | " | " | " | " | " | " | 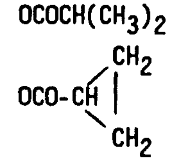 | 531 |
| $C_2H_5OCO-Cl$ | " | " | " | " | " | " | " | $OCO_2C_2H_5$ | 535 |
| $CH_3SO_2-Cl$ | " | " | " | " | " | " | " | $OSO_2CH_3$ | 541 |
| $p-Cl-C_6H_4-SO_2-Cl$ | " | " | " | " | " | " | " | $OSO_2-C_6H_4-p-Cl$ | 638 |

**Tabelle 6c**

Basis-Corticoid = Cortisol (Cort.)

| Reagenz: (Anhydrid oder Chlorid) | Basis-Corticoid | Verf.-Variante Beispiel 2 | A | Y | Z | R(3) | R(2) | R(1) | MS (m/z) M+H$^+$ |
|---|---|---|---|---|---|---|---|---|---|
| $(CH_3CO)_2O$ | Cort. | 2.a.) | CHOH | H | H | H | $-CH_2C(CH_3)_3$ | $OCOCH_3$ | 519 |
| $CH_3CH_2COCl$ | " | 2.b.) | " | " | " | " | " | $OCOC_2H_5$ | 533 |
| $p\text{-}Cl\text{-}C_6H_4\text{-}SO_2\text{-}Cl$ | " | " | " | " | " | " | " | $OSO_2\text{-}C_6H_4\text{-}p\text{-}Cl$ | 652 |

**Tabelle 6d**

Basis-Corticoid = Cortisol (Cort.)

| Reagenz: (Anhydrid oder Chlorid) | Basis-Corticoid | Verf.-Variante Beispiel 2 | A | Y | Z | R(3) | R(2) | R(1) | MS (m/z) $M+H^+$ |
|---|---|---|---|---|---|---|---|---|---|
| $(CH_3CO)_2O$ | Cort. | 2.a.) | CHOH | H | H | H | $CH_2CH_2OCH_3$ | $OCOCH_3$ | 507 |
| $CH_3CH_2COCl$ | " | 2.b.) | " | " | " | " | " | $OCOC_2H_5$ | 521 |
| $CH_3(CH_2)_2COCl$ | " | " | " | " | " | " | " | $OCO(CH_2)_2CH_3$ | 535 |
| 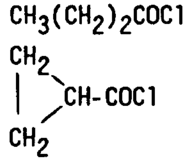 | " | " | " | " | " | " | " | 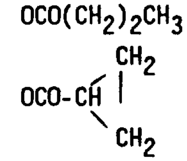 | 533 |
| $C_2H_5OCO-Cl$ | " | " | " | " | " | " | " | $OCO_2C_2H_5$ | 537 |
| $p-Cl-C_6H_4-SO_2-Cl$ | " | " | " | " | " | " | " | $OSO_2-C_6H_4-p-Cl$ | 640 |

**Tabelle 7a**

Basis-Corticoid = Prednison (Pre-n)

| Reagenz: (Anhydrid oder Chlorid) | Basis-Corticoid | Verf.-Variante Beispiel 2 | A | Y | Z | R(3) | R(2) | R(1) | MS (m/z) $M+H^+$ |
|---|---|---|---|---|---|---|---|---|---|
| $(CH_3CO)_2O$ | Pre-n | 2.a.) | C=O | H | H | H | $-CH_2CH(CH_3)_2$ | $OCOCH_3$ | 501 |
| $CH_3CH_2COCl$ | " | 2.b.) | " | " | " | " | " | $OCOC_2H_5$ | 515 |
| $CH_3(CH_2)_2COCl$ | " | " | " | " | " | " | " | $OCO(CH_2)_2CH_3$ | 529 |
| $CH_3(CH_2)_3COCl$ | " | " | " | " | " | " | " | $OCO(CH_2)_3CH_3$ | 543 |
| $(CH_3)_2CHCOCl$ | " | " | " | " | " | " | " | $OCOCH(CH_3)_2$ | 529 |
| $(CH_3)_3CCOCl$ | " | " | " | " | " | " | " | $OCOC(CH_3)_3$ | 543 |
| 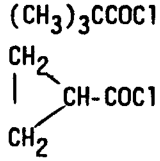 | " | " | " | " | " | " | " | 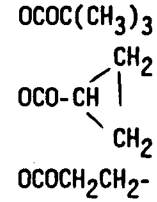 | 527 |
| H $-CH_2CH_2COCl$ | " | " | " | " | " | " | " | $OCOCH_2CH_2-$ H | 583 |
| $C_2H_5OCO-Cl$ | " | " | " | " | " | " | " | $OCO_2C_2H_5$ | 531 |
| $p-Cl-C_6H_4-SO_2-Cl$ | " | " | " | " | " | " | " | $OSO_2-C_6H_4-p-Cl$ | 634 |

**Tabelle 7b**

Basis-Corticoid = Prednison (Pre-n)

| Reagenz: (Anhydrid oder Chlorid) | Basis-Corticoid | Verf.-Variante Beispiel 2 | A | Y | Z | R(3) | R(2) | R(1) | MS (m/z) $M+H^+$ |
|---|---|---|---|---|---|---|---|---|---|
| $(CH_3CO)_2O$ | Pre-n | 2.a.) | C=O | H | H | H | $-CH(CH_3)_2$ | $OCOCH_3$ | 487 |
| $CH_3CH_2COCl$ | " | 2.b.) | " | " | " | " | " | $OCOC_2H_5$ | 501 |
| $CH_3(CH_2)_2COCl$ | " | " | " | " | " | " | " | $OCO(CH_2)_2CH_3$ | 515 |
| $CH_3(CH_2)_3COCl$ | " | " | " | " | " | " | " | $OCO(CH_2)_3CH_3$ | 529 |
| $(CH_3)_2CHCOCl$ | " | " | " | " | " | " | " | $OCOCH(CH_3)_2$ | 515 |
| $(CH_3)_3CCOCl$ | " | " | " | " | " | " | " | $OCOC(CH_3)_3$ | 529 |
| 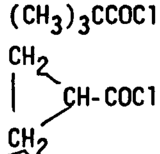 | " | " | " | " | " | " | " | 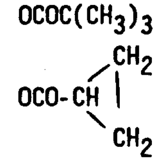 | 513 |
| 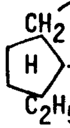 $-CH_2CH_2COCl$ | " | " | " | " | " | " | " | $OCOCH_2CH_2-$ H | 569 |
| $C_2H_5-OCO-Cl$ | " | " | " | " | " | " | " | $OCO_2C_2H_5$ | 517 |
| $p-Cl-C_6H_4-SO_2-Cl$ | " | " | " | " | " | " | " | $OSO_2-C_6H_4-p-Cl$ | 620 |

**Tabelle 7c**

Basis-Corticoid = Prednison (Pre-n)

| Reagenz: · (Anhydrid oder Chlorid) | Basis-Corticoid | Verf.-Variante Beispiel 2 | A | Y | Z | R(3) | R(2) | R(1) | MS (m/z) $M+H^+$ |
|---|---|---|---|---|---|---|---|---|---|
| $(CH_3CO)_2O$ | Pre-n | 2.a.) | C=O | H | H | H | $-CH_2C(CH_3)_3$ | $OCOCH_3$ | 515 |
| $CH_3CH_2COCl$ | " | 2.b.) | " | " | " | " | " | $OCOC_2H_5$ | 529 |
| 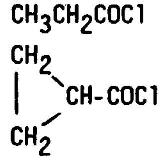 | " | " | " | " | " | " | " | 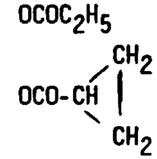 | 541 |
| $C_2H_5OCO-Cl$ | " | " | " | " | " | " | " | $OCO_2C_2H_5$ | 545 |
| $p-Cl-C_6H_4-SO_2-Cl$ | " | " | " | " | " | " | " | $OSO_2-C_6H_4-p-Cl$ | 648 |

**Tabelle 7d**

Basis-Corticoid = Prednison (Pre-n)

| Reagenz: (Anhydrid oder Chlorid) | Basis-Corticoid | Verf.-Variante Beispiel 2 | A | Y | Z | R(3) | R(2) | R(1) | MS (m/z) $M+H^+$ |
|---|---|---|---|---|---|---|---|---|---|
| $(CH_3CO)_2O$ | Pre-n | 2.a.) | C=O | H | H | H | $CH_2CH_2OCH_3$ | $OCOCH_3$ | 503 |
| $CH_3CH_2COCl$ | " | 2.b.) | " | " | " | " | " | $OCOC_2H_5$ | 517 |
| 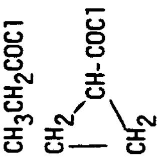 | " | " | " | " | " | " | " | 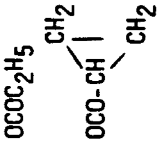 | 529 |
| $p-Cl-C_6H_4-SO_2-Cl$ | " | " | " | " | " | " | " | $OSO_2-C_6H_4-p-Cl$ | 637 |

**Beispiel 6**

Eine Lösung von 540 mg Prednisolon-17-isobutylcarbonat-21-p-Chlorbenzolsulfonat in 5,6 ml absolutem Dimethylformamid wird mit 1,04 g getrocknetem Lithiumchlorid versetzt und in $N_2$-Atmosphäre 4 Std. bei 100 °C gerührt. man gießt in 200 ml wässrige Kochsalzlösung ein, filtriert den gebildeten Niederschlag ab, trocknet ihn und kristallisiert ihn aus

Aceton/Methylenchlorid/Diethylether aus. Man erhält 420 mg Prednisolon-17-isobutylcarbonat-21-chlorid.
Schmp. 132 °C
MS : m/z = 480 [M + H$^+$]
DC : 0,7

In gleicher Weise werden aus Prednisolon-17-isopropyl-carbonat-21-p-chlorbenzolsulfonat das Prednisolon-17-iso-propylcarbonat-21-chlorid, aus Prednisolon-17-tert.-butyl-methylcarbonat-21-p-chlorbenzolsulfonat das Prednisolon-17-tert.-butylmethylcarbonat-21-chlorid, aus Prednisolon-17-methoxyethylcarbonat-p-chlorbensolsulfonat das Prednisolon-17-methoxyethylcarbonat-21-chlorid erhalten.

**Beispiel 7**

Eine Lösung von 300 mg Prednisolon-17-isobutylcarbonat-21-p-chlorbensolsulfonat in 3 ml absolutem Dimethylformamid wird mit 630 mg trockenem Lithiumbromid versetzt und unter $N_2$-Atmosphäre 4 Std. bei 110 °C, gerührt. Man gießt in 20 ml wässrige Kochsalzlösung ein, filtriert den gebildeten Niederschlag ab, trocknet ihn und chromatographiert ihn an Kieselgel (Säulenmaße 17 x 3 cm) mit Methylenchlorid/Methanol 99:1 als Eluationsmittel. Nach dem Abdestillieren der Lösungsmittel werden nach Kristallisation aus Diethylether 310 mg Prednisolon-17-isobutylcarbonat-21-bromid erhalten:
MS : m/z = 524 [M + H$^+$]

Zum gleichen Reaktionsprodukt gelangt man, wenn man anstelle von LiBr 0,5 g. Kaliumbromid in die Reaktion einsetzt, weiterbehandelt, aufarbeitet und reindarstellt (Chromatographie).

In gleicher Weise werden aus Prednisolon-17-isopropylcarbonat-21-p-chlorbenzolsulfanat das Prednisolon-17-isopropylcarbonat-21-bromid, aus Prednisolon-17-tert.-butylmethylcarbonat-21-chlorbenzol-sulfonat das Prednisolon-17-tert.-butylmethylcarbonat-21-bromid, aus Prednisolon-17-methoxyethylcarbonat-p-chlorbenzolsulfonat, das Prednisolon-17-methoxyethylcarbonat-21-bromid erhalten.

**Beispiel 8**

Eine Lösung von 540 mg Prednisolon-17-isobutylcarbonat-21-p-chlorbenzolsulfonat in 10 ml absol. Dimethylformamid wird mit 1,00 g getrocknetem Kaliumjodid versetzt und in $N_2$-Atmosphäre 2 Std. bei 100 °C gerührt. Man gießt in 200 ml wässrige Kochsalzlösung ein, filtriert einen gebildeten Niederschlag ab, trocknet ihn und kristallisiert ihn aus Aceton/Methylenchlorid/Diethylether aus oder chromatographiert ihn, wie in Beispiel 7 für das entsprechende 21-Bromid angegeben. Man erhält 300 mg Prednisolon-17-isobutylcarbonat-21-jodid.
Schmp. 110 °C
MS : m/z = 571 [M + H$^+$]
DC : 0,7

In gleicher Weise werden aus Prednisolon-17-isopropylcarbonat-21-p-chlorbenzolsulfonat das Prednisolon-17-isopropylcarbonat-21-jodid, aus Prednisolon-17-tert.-butylmethylcarbonat-21-p-chlorbenzol-sulfonat das Prednisolon-17-tert.-butylmethylcarbonat-21-jodid, aus Prednisolon-17-methoxyethylcarbonat-p-chlorbenzol-sulfonat das Prednisolon-17-methoxyethylcarbonat-21-jodid erhalten.

Zu den gleichen Reaktionsprodukten gelangt man, wenn man 300 mg eines o.a. 21-Chlorsulfonats mit 60 mg Lithiumjodid in 6 ml aboslutem Aceton oder Butanol-(2) 3 Std. unter $N_2$ am Rückfluß kocht, in 20 ml $H_2O$ eingießt, mit Methylenchlorid extrahiert und nach üblicher Aufarbeitung aus Diisopropylether kristallisiert (200-300 mg Ausbeute).

**Beispiel 9**

Gemäß Tabelle 8 erhält man mit Alkylihalogeniden aus den zugrundeliegenden Corticoid-17-alkyl- bzw. 17-methoxyethyl-carbonat-21-p-chlor-benzolsulfonaten die entsprechenden 21-Halogenidderivate der Formel I, wenn man die Reaktionen in der Weise, wie zu den Beispielen 6, 7 und 8 beschrieben, durchführt:
Die Charakterisierung der Reaktionsprodukte kann durch Dünnschichtchromatographie (DC) erfolgen; hierbei haben die Reaktionsprodukte $R_F$-Werte von etwa 0,65-0,75. In der Regel werden die Reaktionsprodukte durch Massenspektren mit MS = m/z = .... (M + H$^+$) charakterisiert (In der Regel FAB-Spektren).
Die M + H$^+$-Werte wurden jeweils aufgerundet. Auch IR-, $^1$H-NMR- und UV-Spektren können zur Charakterisierung herangezogen werden.

**Tabelle 8** (Abkürzung für Basis-Corticoide siehe Tabellen 2-7)

| Reagenz | Darst. gemäß Beispiel | Basis-Corticoid | A | Y | Z | R(3) | R(2) | R(1) |
|---|---|---|---|---|---|---|---|---|
| LiCl | 6 | Dex | CHOH | F | H | $CH_3$ | $-CH(CH_3)_2$ | Cl |
| LiBr | 7 | " | " | " | " | " | " | Br |
| KJ | 8 | " | " | " | " | " | " | J |
| LiCl | 6 | M-pred | CHOH | H | $CH_3$ | H | " | Cl |
| LiBr | 7 | " | " | " | " | " | " | Br |
| KJ | 8 | " | " | " | " | " | " | J |
| LiCl | 6 | F-Dex | CHOH | F | F | $CH_3$ | " | Cl |
| LiBr | 7 | " | " | " | " | " | " | Br |
| KJ | 8 | " | " | " | " | " | " | J |
| LiCl | 6 | Cort. | CHOH | H | H | H | " | Cl |
| LiBr | 7 | " | " | " | " | " | " | Br |
| LIJ | 8 | " | " | " | " | " | " | J |
| LiCl | 6 | Pre-n | C=O | H | H | H | " | Cl |
| LiBr | 7 | " | " | " | " | " | " | Br |
| LiJ | 8 | " | " | " | " | " | " | J |

**Tabelle 8** (Abkürzung für Basis-Corticoide siehe Tabellen 2-7)

Fortsetzung 1

| Reagenz | Darst. gemäß Beispiel | Basis-Corticoid | A | Y | Z | R(3) | R(2) | R(1) |
|---|---|---|---|---|---|---|---|---|
| LiCl | 6 | Dex | CHOH | F | H | $CH_3$ | $-CH_2CH(CH_3)_2$ | Cl |
| LiBr | 7 | " | " | " | " | " | " | Br |
| KJ | 8 | " | " | " | " | " | " | J |
| LiCl | 6 | M-pred | CHOH | H | $CH_3$ | H | " | Cl |
| LiBr | 7 | " | " | " | " | " | " | Br |
| KJ | 8 | " | " | " | " | " | " | J |
| LiCl | 6 | F-Dex | CHOH | F | F | $CH_3$ | " | Cl |
| LiBr | 7 | " | " | " | " | " | " | Br |
| KJ | 8 | " | " | " | " | " | " | J |
| LiCl | 6 | Cort. | CHOH | H | H | H | " | Cl |
| LiBr | 7 | " | " | " | " | " | " | Br |
| LIJ | 8 | " | " | " | " | " | " | J |
| LiCl | 6 | Pre-n | C=O | H | H | H | " | Cl |
| LiBr | 7 | " | " | " | " | " | " | Br |
| LiJ | 8 | " | " | " | " | " | " | J |

**Tabelle 8** (Abkürzung für Basis-Corticoide siehe Tabellen 2-7)

Fortsetzung 2

| Reagenz | Darst. gemäß Beispiel | Basis-Corticoid | A | Y | Z | R(3) | R(2) | R(1) |
|---|---|---|---|---|---|---|---|---|
| LiCl | 6 | Dex | CHOH | F | H | $CH_3$ | $-CH_2C(CH_3)_3$ | Cl |
| LiBr | 7 | " | " | " | " | " | " | Br |
| KJ | 8 | " | " | " | " | " | " | J |
| LiCl | 6 | M-pred | CHOH | H | $CH_3$ | H | " | Cl |
| LiBr | 7 | " | " | " | " | " | " | Br |
| KJ | 8 | " | " | " | " | " | " | J |
| LiCl | 6 | F-Dex | CHOH | F | F | $CH_3$ | " | Cl |
| LiBr | 7 | " | " | " | " | " | " | Br |
| KJ | 8 | " | " | " | " | " | " | J |
| LiCl | 6 | Cort. | CHOH | H | H | H | " | Cl |
| LiBr | 7 | " | " | " | " | " | " | Br |
| LIJ | 8 | " | " | " | " | " | " | J |
| LiCl | 6 | Pre-n | C=O | H | H | H | " | Cl |
| LiBr | 7 | " | " | " | " | " | " | Br |
| LiJ | 8 | " | " | " | " | " | " | J |

**Tabelle 8** (Abkürzung für Basis-Corticoide siehe Tabellen 2-7)

Fortsetzung 3

| Reagenz | Darst. gemäß Beispiel | Basis-Corticoid | A | Y | Z | R(3) | R(2) | R(1) |
|---|---|---|---|---|---|---|---|---|
| LiCl | 6 | Dex | CHOH | F | H | $CH_3$ | $-CH_2CH_2OCH_3$ | Cl |
| LiBr | 7 | " | " | " | " | " | " | Br |
| KJ | 8 | " | " | " | " | " | " | J |
| LiCl | 6 | M-pred | CHOH | H | $CH_3$ | H | " | Cl |
| LiBr | 7 | " | " | " | " | " | " | Br |
| KJ | 8 | " | " | " | " | " | " | J |
| LiCl | 6 | F-Dex | CHOH | F | F | $CH_3$ | " | Cl |
| LiBr | 7 | " | " | " | " | " | " | Br |
| KJ | 8 | " | " | " | " | " | " | J |
| LiCl | 6 | Cort. | CHOH | H | H | H | " | Cl |
| LiBr | 7 | " | " | " | " | " | " | Br |
| LIJ | 8 | " | " | " | " | " | " | J |
| LiCl | 6 | Pre-n | C=O | H | H | H | " | Cl |
| LiBr | 7 | " | " | " | " | " | " | Br |
| LiJ | 8 | " | " | " | " | " | " | J |

**Beispiel 10**

Zu einer Lösung von 1,5 g Prednisolon in 45 ml absolutem Dioxan gibt man 7 ml Orthokolensäuretetraisopropylether sowie 0,2 g p-Toluolsulfonsäure. Nach 3 bis 5 Std. Erhitzen auf 60 °C und Rühren zeigt ein DC-Diagramm einen intensiven Fleck mit dem $R_F$-Wert von ca. 0,8 für das erwartete Reaktionsprodukt und

nur noch einen äußerst schwach sichtbaren "Fleck" für das Ausgangsprodukt mit dem $R_F$-Wert bei ca. 0,3. Man gießt das Reaktionsgemisch in 500 ml wässrige Kochsalzlösung ein, filtriert den Niederschlag ab, wäscht ihn mit Wasser und trocknet ihn. Nach dem Umkristallisieren aus Methylenchlorid/Ethanol/Diethylether erhält man 1,3 g Prednisolon-17,21-di-isopropyl-ortho-carbonat vom Schmp. 185 °C.
MS = m/z = 489 ($M+H^+$)
IR = 3420, 1730, 1660, 1620, 1600, 1100 $cm^{-1}$
UV: $\lambda_{max}$ = 242,5 nm, $\epsilon$ = 15600.

Wiederholung des Ansatzes, der aber jetzt anstatt auf 60 °C zu erwärmen, 3 Std. bei 20 °C (Zimmertemp.) gerührt wird, ergibt nach dem analogen Aufarbeiten ein Reaktionsprodukt (1,1 g), das in allen spektralen Daten (z.B. MS = M + $H^+$ = 360) mit dem Ausgangsmaterial Prednisolon weitgehend identisch ist.
DC: $R_F$ = 0,3 = sehr starker Fleck (= Prednisolon),
$R_F$ etwa 0,8 nur sehr schwacher Fleck sichtbar
(= gewünschtes Reaktionsprodukt)

**Beispiel 11**

Zu einer Lösung von 25 g Prednisolon in 750 ml absolutem Dioxan gibt man 70 ml Orthokohlensäuretetraisobutylether sowie 2 g p-Toluolsulfonsäure. Nach 5 Std. Erhitzen auf 60 °C und Rühren zeigt im DC-Diagramm einen intensiven Fleck mit dem $R_F$-Wert von ca. 0,8 für das erwartete Reaktionsprodukt und nur noch einen äußerst schwach sichtbaren "Fleck" für das Ausgangsprodukt mit dem $R_F$-Wert bei ca. 0,3. Man gießt das Reaktionsgemisch in 8 l wässrige Kochsalzlösung ein, filtriert den Niederschlag ab, wäscht ihn mit Wasser und trocknet ihn. Nach dem Umkristallisieren aus Methylenchlorid/Ethanol/Diethylether erhält man 38,8 g Prednisolon-17,21-diisobutyl-orthocarbonat vom Schmp. 113 °C.
MS = m/z = 517 ($M+H^+$)
IR = 3380, 1750, 1735, 1660, 1615, 1600, 1120, 1090 $cm^{-1}$

**Beispiel 12**

Zu einer Lösung von 25 g Prednisolon in 550 ml absolutem Dioxan gibt man 60 ml Orthokohlensäuretetra-tert.-butylmethylester sowie 2 g p-Toluolsulfonsäure. Nach 6 Std. Erhitzen auf 60 °C und Rühren zeigt ein DC-Diagramm einen intensiven Fleck mit dem $R_F$-Wert von ca. 0,8 für das erwartete Reaktionsprodukt und nur noch einen äußerst schwach sichtbaren "Fleck" für das Ausgangsprodukt mit dem $R_F$-Wert bei ca. 0,3. Man gießt das Reaktionsgemisch in 8 l wässrige Kochsalzlösung ein, filtriert den öligen Niederschlag ab, nimmt ihn in Methylenchlorid auf, wäscht die organische Phase mit Wasser, trocknet sie und destilliert das Lösungsmittel ab. Das Öl (35 g) wird an Aluminiumoxid, neutral, Akt-St. II (Säule 25 x 8 cm) mit Methylenchlorid als Eluationsmittel chromatographiert (100 ml Fraktionen). Die Fraktionen, die im DC einen $R_F$-Wert von etwa 0,8 cm zeigen, werden vereinigt, vom Eluationsmittel durch Abdestillieren befreit und ergeben nach Vereinigung 14 g gelbliches öliges Prednisolon-17,21-di-tert.-butylmethyl-orthocarbonat.
MS: m/z = 545 ($M+H^+$)

**Beispiel 13**

Zu einer Lösung von 25 g Prednisolon in 500 ml absolutem Dioxan gibt man 7 ml Orthokohlensäuretetra-methoxyethylester sowie 2 g p-Toluolsulfonsäure. Nach 5 Std. Erhitzen auf 60 °C und Rühren zeigt ein DC-Diagramm einen intensiven Fleck mit dem $R_F$-Wert von ca. 0,8 für das erwartete Reaktionsprodukt und nur noch einen äußerst schwach sichtbaren "Fleck" für das Ausgangsprodukt mit dem $R_F$-Wert bei ca. 0,3. Man gießt das Reaktionsgemisch in 8 l wässrige Kochsalzlösung ein und filtiert vom öligen Niederschlag ab. Weiterbehandlung und chromatographische Reindarstellung wie bei Beispiel 12. Man erhält 27,5 g Prednisolon-17,21-di-methoxyethyl-orthocarbonat vom Schmp. 125 °C (Zers.) (durch Anreiben mit Diethylether) MS: m/z = 521 ($M+H^+$)
IR = 3400, 1725, 1660, 1620, 1600, 1125, 1060 $cm^{-1}$

**Beispiel 14**

In gleicher Weise, wie in Beispiel 10 beschrieben, werden erhalten, wenn man anstelle von Prednisolon

einsetzt:
aus Dexamethason das
Dexamethason-17,21-di-isopropyl-orthocarbonat,
MS: m/z = 521 $(M+H^+)$,
aus 6α-Methylprednisolon das
6α-Methylprednisolon-17,21-di-isopropyl-orthocarbonat,
MS: m/z = 503 $(M+H^+)$,
aus 6α-Fluordexamethason das
6α-Fluordexamethanon-17,21-di-isopropyl-othocarbonat,
MS: m/z = 539 $(M+H^+)$ ,
aus Cortisol das
Cortisol-17,21-di-isopropylorthocarbonat,
MS: m/z = 491 $(M+H^+)$,
aus Prednison das
Prednison-17,21-di-isopropylorthocarbonat,
MS: m/z = 487 $(M+H^+)$,
aus Cortison das
Cortison-17,21-di-isopropylorthocarbonat,
MS: m/z = 489 $(M+H^+)$,
aus Reichsteins Substanz S das
[Reichsteins Substanz S]-17,21-di-isopropylorthocarbonat,
MS: m/z = 475 $(M+H^+)$,
aus 6α,16α-Dimethylprednisolon das
6α,16α-Dimethylprednisolon-17,21-di-isopropylorthocarbonat,
MS: m/z = 517 $(M+H^+)$,
aus 6α-Fluorprednisolon das
6α-Fluor-prednisolon-17,21-di-isopropylorthocarbonat,
MS: m/z = 507 $(M+H^+)$,

**Beispiel 15**

In gleicher Weise, wie in Beispiel 11 beschrieben, werden erhalten, wenn man anstelle von Prednisolon einsetzt:
aus Dexamethason das
Dexamethason-17,21-di-isobutyl-orthocarbonat,
MS: m/z 549 $(M+H^+)$,
aus 6α-Methylprednisolon das
6α-Methylprednisolon-17,21-di-isobutyl-orthocaronat,
MS: m/z = 531 $(M+H^+)$,
aus 6α-Fluordexamethason das
6α-Fluordexamethason-17,21-di-isobutyl-orthocarbonat,
MS: m/z = 567 $(M+H^+)$,
aus Cortisol das
Cortisol-17,21-di-isobutyl-orthocarbonat,
MS: m/z = 519 $(M+H^+)$,
aus Prednison das
Prednison-17,21-di-iso-butylorthocarbonat,
MS: m/z = 515 $(M+H^+)$,
aus Cortison das
Cortison-17,21-di-iso-butylorthocarbonat,
MS: m/z = 517 $(M+H^+)$,
aus Reichsteins Substanz S das
[Reichsteins Substanz S]-17,21-di-isobutylorthocarbonat,
MS: m/z = 503 $(M+H^+)$,
aus 6α,16α-Dimethylprednisolon das
6α,16α-Dimethylprednisolon-17,21-di-isobutylorthocarbonat,
MS: m/z = 545 $(M+H^+)$,
aus 6α-Fluor-prednisolon das

6α-Fluorprednisolon-17,21-di-isobutyl-orthocarbonat,
MS: m/z = 535 ($M+H^+$),
aus
6α-Methyl-dexamethason das
6α-Methyl-dexamethason-di-isobutyl-orthocarbonat,

**Beispiel 16**

In gleicher Weise, wie in Beispiel 12 beschrieben, werden erhalten, wenn man anstelle von Prednisolon einsetzt:
aus Dexamethason das
Dexamethason-17,21-di-tert.-butylmethyl-orthocarbonat,
MS: m/z = 577 ($M+H^+$),
aus 6α-Methylprednisolon das
6α-Methylprednisolon-17,21-di-tert.-butylmethyl-orthocarbonat,
MS: m/z = 559 ($M+H^+$),
aus 6α-Fluordexamethason das
6α-Fluordexamethason-17,21-di-tert.-butylmethyl-orthocarbonat,
MS: m/z = 595 ($M+H^+$),
aus Cortisol das
Cortisol-17,21-di-tert.-butylmethyl-orthocarbonat,
MS: m/z = 547 ($M+H^+$),
aus Prednison das
Prednison-17,21-di-tert.-butylmethyl-orthocarbonat,
MS: m/z = 543 ($M+H^+$),

**Beispiel 17**

In gleicher Weise, wie in Beispiel 13 beschrieben, werden erhalten, wenn man anstelle von Prednisolon einsetzt:
aus Dexamethason das
Dexamethason-17,21-di-methoxyethyl-orthocarbonat,
MS: m/z = 553 ($M+H^+$),
aus 6α-Methylprednisolon das
6α-Methylprednisolon-17,21-di-methoxyethyl-orthocarbonat,
MS: m/z 535 ($M+H^+$),
aus 6α-Fluordexamethason das
6α-Fluordexamethason-17,21-di-methoxyethyl-ortbocarbonat,
MS: m/z ($M+H^+$),
aus Cortisol das
Cortisol-17,21-di-methoxyethyl-orthocarbonat,
MS: m/z = 523 ($M+H^+$),
aus Prednison das
Prednison-17,21-di-methoxyethyl-orthocarbonat
MS: m/z = 519 ($M+H^+$).

**Patentansprüche**

1. Corticoid-17-alkylcarbonate der Formel I

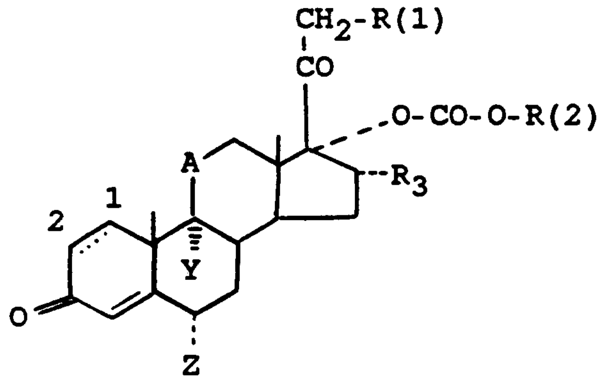

I,

in welcher bedeuten:

A = CHOH in beliebiger sterischer Anordnung, $CH_2$, C = O,

Y = Wasserstoff, Fluor, Chlor,

Z = Wasserstoff, Fluor, Methyl

R(1) = F, Cl, Br, J, O-Acyl der Formel II: $-O-CO-(CH_2)_n-R(4)$, Oxycarbonyloxyalkyl der Formel III: $-O-CO-O-(CH_2)_n-R(4)$, Sulfonsäurealkyl-, Sulfonsäurearylester der Formel IV: $-O-SO_2-R(5)$ mit R(4) gleich Wasserstoff, $(C_1-C_{10})$-Alkyl, $(C_3-C_6)$-Cycloalkyl oder, wenn n = 1 ist, Fluor, Chlor, Brom,

R(5) gleich $(C_1-C_4)$-Alkyl, Phenyl, Chlorphenyl, Methylphenyl, n eine ganze Zahl von Null bis 4,

R(2) = verzweigtes $(C_3-C_8)$-Alkyl, $-(CH_2)_{2-4}-OCH_3$

R(3) = Wasserstoff, Methyl.

**2.** Corticoid-17-alkylcarbonate nach Anspruch 1, dadurch gekennzeichnet, daß

R(1), A, Y, Z, R(3) und R(4) wie in Anspruch 1 definiert sind und daß

R(2) verzweigtes $(C_3-C_5)$-Alkyl oder $(CH_2)_2-OCH_3$ und

R(5) Methyl, Ethyl, Propyl oder Phenyl ist, das unsubstituiert oder in p-Stellung durch Chlor oder Methyl substituiert ist,

sind.

**3.** Verfahren zum Herstellen einer Verbindung I nach Anspruch 1, dadurch gekennzeichnet, daß man Verbindungen der Formel V.

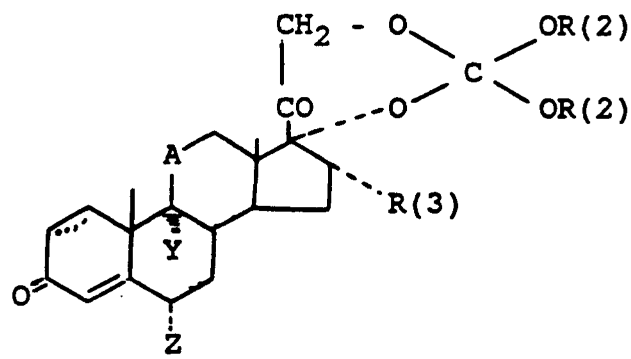

V

mit einer schwachen Säure hydrolysiert und die erhaltene 21-Hydroxy-Verbindung der Formel I mit $R_1$ = OH mit einem Halogenid oder Anhydrid einer Carbonsäure der Formel VI

$R(4)-(CH_2)_n-COOH$ VI

oder einem Halogenformat der Formel VII

$R(4)-(CH_2)_n-OCO-Halogen$

oder einem Sulfonsäurehalogenid der Formel VIII

$R(5)-SO_2-Halogen$ VIII

verestert
und gegebenenfalls den so erhaltenen 21-Sulfonsäureester mit Halogenidsalzen zu 21-Halogeniden der Formel I mit R(1) gleich Chlor, Brom, Jod, Fluor umsetzt.

**4.** Verfahren zum Herstellen einer Verbindung V

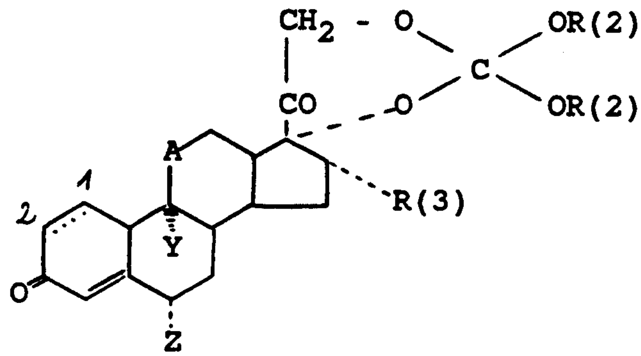

V,

dadurch gekennzeichnet, daß man eine Verbindung der Formel VI

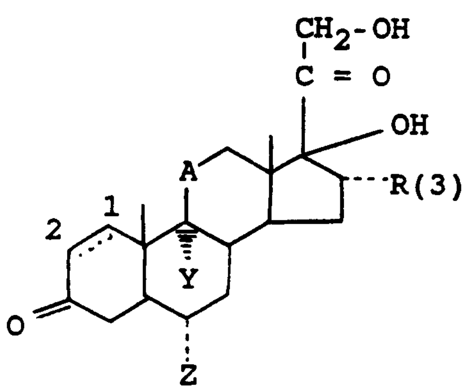

VI,

in der A, Y, Z, R(3) und 2 --- 1 wie in Anspruch 1 definiert sind,
mit im Alkylanteil verzweigten oder substituierten Tetraalkyl-orthocarbonaten der Formel VII

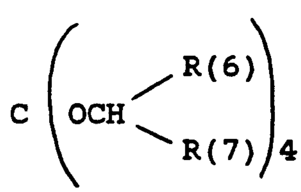

VII

in der

R(6) = H, $CH_3$ und
R(7) = $CH_3$, $CH(CH_3)_2$, $C(CH_3)_3$ oder $CH_2OCH_3$ sind, wobei R(6) jedoch dann nicht Wasserstoff bedeutet, wenn R(7) eine unverzweigte Kohlenstoffkette enthält, in inerten Lösungsmitteln bei Temperaturen von größer als 20° bis 120 °C, vorzugsweise bis zum Siedepunkt der Reaktionsgemische, insbesondere bei etwa 50° bis 60 °C, umsetzt.

**5.** Medikament zur Behandlung von Dermatosen insbesondere entzündlichen und allergischen, gekennzeichnet durch einen wirksamen Gehalt einer Verbindung I nach Anspruch 1.

**6.** Verfahren zum Behandeln von Dermatosen, dadurch gekennzeichnet, daß man eine wirksame Menge einer Verbindung I nach Anspruch 1, kombiniert mit pharmazeutisch üblichen Zuschlagstoffen, auf die befallene Hautstelle aufbringt.

**7.** Verwendung einer Verbindung I nach Anspruch 1 zum Herstellen eines Medikaments zur Behandlung von Dermatosen.

**Patentansprüche für folgende Vertragsstaaten: ES, GR**

**1.** Verfahren zum Herstellen einer Verbindung I

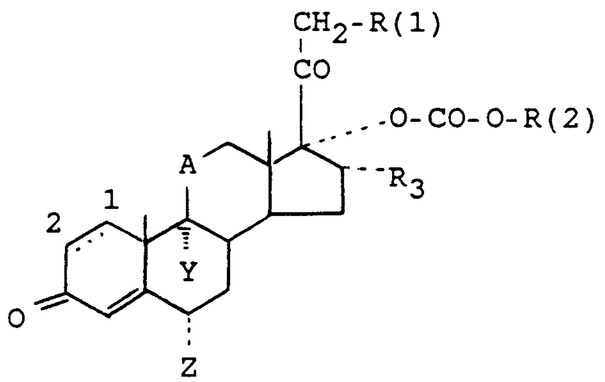

I,

in welcher bedeuten:

A = CHOH in beliebiger sterischer Anordnung, $CH_2$, C=O,
Y = Wasserstoff, Fluor, Chlor,
Z = Wasserstoff, Fluor, Methyl
R(1) = F, Cl, Br, J, O-Acyl der Formel II: $-O-CO-(CH_2)_n-R(4)$, Oxycarbonyloxyalkyl der Formel III: $-O-CO-O-(CH_2)_n-R(4)$, Sulfonsäurealkyl-, Sulfonsäurearylester der Formel IV: $-O-SO_2-R(5)$ mit
R(4) gleich Wasserstoff, $(C_1-C_{10})$-Alkyl, $(C_3-C_6)$-Cycloalkyl oder, wenn n = 1 ist, Fluor, Chlor, Brom,

R(5) gleich $(C_1-C_4)$-Alkyl, Phenyl, Chlorphenyl, Methylphenyl,
n eine ganze Zahl von Null bis 4,
R(2) = verzweigtes $(C_3-C_8)$-Alkyl, $-(CH_2)_{2-4}-OCH_3$

R(3) = Wasserstoff, Methyl, dadurch gekennzeichnet, daß man Verbindungen der Formel V

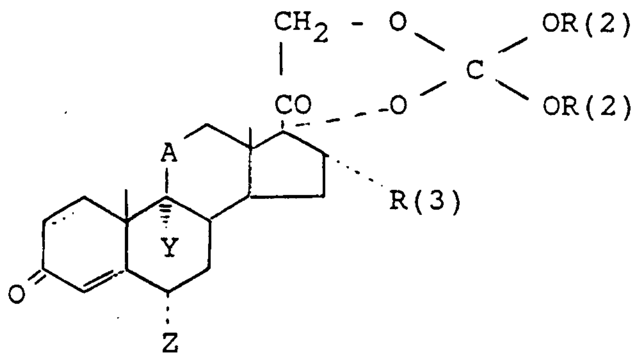

V

mit einer schwachen Säure hydrolysiert und die erhaltene 21-Hydroxy-Verbindung der Formel I mit $R_1$ = OH mit einem Halogenid oder Anhydrid einer Carbonsäure der Formel VI

$R(4)-(CH_2)_n-COOH$ VI

oder einem Halogenformat der Formel VII

$R(4)-(CH_2)_n-OCO$-Halogen

oder einem Sulfonsäurehalogenid der Formel VIII

$R(5)-SO_2$-Halogen VIII

verestert
und gegebenenfalls den so erhaltenen 21-Sulfonsäureester mit Halogenidsalzen zu 21-Halogeniden der Formel I mit R(1) gleich Chlor, Brom, Jod, Fluor umsetzt.

**2.** Verfahren nach Anspruch 1, dadurch gekennzeichnet, daß
R(1), A, Y, Z, R(3) und R(4) wie in Anspruch 1 definiert sind und daß
R(2) verzweigtes $(C_3-C_5)$-Alkyl oder $(CH_2)_2-OCH_3$ und
R(5) Methyl, Ethyl, Propyl oder Phenyl ist, das unsubstituiert oder in p-Stellung durch Chlor oder Methyl substituiert ist,
sind.

**3.** Verfahren nach Anspruch 1, dadurch gekennzeichnet, daß man eine Verbindung der Formel VI

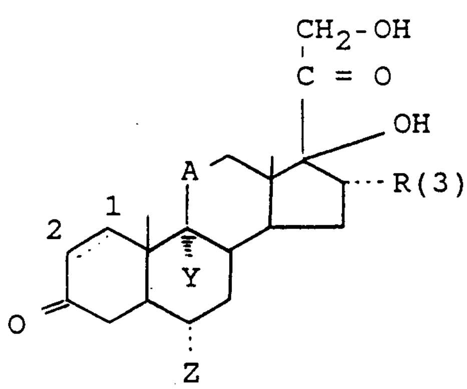

VI,

in der A, Y, Z, R(3) und 2 --- 1 wie oben definiert sind, mit im Alkylanteil verzweigten oder substituierten Tetraalkyl-orthocarbonaten der Formel VII

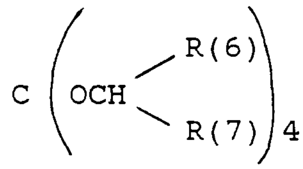

VII,

in der
R(6) = H, $CH_3$ und
R(7) = $CH_3$, $CH(CH_3)_2$, $C(CH_3)_3$ oder $CH_2OCH_3$ sind, wobei R(6) jedoch dann nicht Wasserstoff bedeutet, wenn R(7) eine unverzweigte Kohlenstoffkette enthält, in inerten Lösungsmitteln bei Temperaturen von größer als 20° bis 120 °C, vorzugsweise bis zum Siedepunkt der Reaktionsgemische, insbesondere bei etwa 50° bis 60 °C, umsetzt
und daß man danach die Verbindungen V

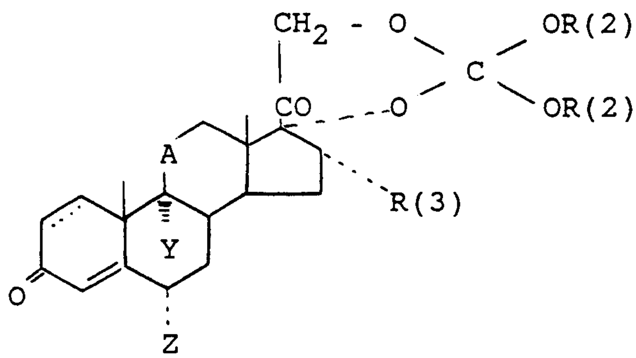

V

mit einer schwachen Säure hydrolysiert und die erhaltene 21-Hydroxy-Verbindung der Formel I mit $R_1$ = OH mit einem Halogenid oder Anhydrid einer Carbonsäure der Formel VI

$R(4)-(CH_2)_n-COOH$ VI

oder einem Halogenformat der Formel VII

$R(4)-CH_2)_n-OCO-Halogen$

oder einem Sulfonsäurehalogenid der Formel VIII

$R(5)-SO_2-Halogen$ VIII

verestert
und gegebenenfalls den so erhaltenen 21-Sulfonsäureester mit Halogenidsalzen zu 21-Halogeniden der Formel I mit R(1) gleich Chlor, Brom, Jod, Fluor umsetzt.

**4.** Verfahren zum Herstellen eines Medikaments zur Behandlung von Dermatrosen, dadurch gekennzeichnet, daß man eine wirksame Menge einer nach Anspruch 1 erhaltenen Verbindung mit pharmazeutisch üblichen Zuschlagstoffen versieht.